# EUROPEAN PATENT SPECIFICATION

(11) **EP 3 553 055 B1**
(45) Date of publication and mention of the grant of the patent: **13.07.2022**
(21) Application number: 18167343.5
(22) Date of filing: 13.04.2018
(51) Int. Cl.: C07D 317/12, C07D 319/06, C07D 321/06, C11B 9/00

(54) **CYCLIC ACETALS OF 2-PHENYL-C3-C5-ALKANALS AND STEREOISOMERS THEREOF FOR USE AS AROMA CHEMICALS**
CYCLISCHE ACETALE VON 2-PHENYL-C3-C5-ALKANALEN UND DEREN STEREOISOMERE ZUR VERWENDUNG ALS AROMASTOFFE
ACETALS CYCLIQUES DES 2-PHENYL-C3-C5-ALKANALS ET LEURS STÉRÉOISOMERES ET LEUR UTILISATION COMME AGENTS CHIMIQUES AROMATIQUES

(43) Date of publication of application: 16.10.2019
(73) Proprietor: BASF SE, 67056 Ludwigshafen am Rhein (DE)
(72) Inventor: BRU ROIG, Miriam, 67056 Ludwigshafen (DE); STEGMANN, Veit, 67056 Ludwigshafen (DE); PELZER, Ralf, 68623 Lampertheim (DE); DANZ, Manuel, 67056 Ludwigshafen (DE); GARLICHS, Florian, 68623 Lampertheim (DE)

(56) References cited:
- JP-A- H05 230 496
- US-A- 1 837 273
- Makoto Shibagaki: "Convenient Preparation of Acetals over Hydrous Zirconium Oxide", Bulletin of the Chemical Society of Japan, 1 April 1990 (1990-04-01), pages 1258-1259, XP055480656, Retrieved from the Internet: URL:http://www.journal.csj.jp/doi/pdf/10.1 246/bcsj.63.1258 [retrieved on 2018-06-01]
- PANDUR VENKATESAN BALAJI ET AL: "Stereoselective Anti-Markovnikov Geminal Diamination and Dioxy-gena-tion of Vinylarenes Mediated by the Bromonium Ion", EUROPEAN JOURNAL OF ORGANIC CHEMISTRY, vol. 2016, no. 14, 26 April 2016 (2016-04-26), pages 2547-2554, XP055480658, ISSN: 1434-193X, DOI: 10.1002/ejoc.201600203
- MASANARI KIMURA ET AL: "Pd(0)-Catalyzed Amphiphilic Allylation of Aldehydes with Vinyl Epoxide", JOURNAL OF THE AMERICAN CHEMICAL SOCIETY, vol. 129, no. 14, 1 April 2007 (2007-04-01), pages 4122-4123, XP055481792, US ISSN: 0002-7863, DOI: 10.1021/ja0687320
- DATABASE CA [Online] CHEMICAL ABSTRACTS SERVICE, COLUMBUS, OHIO, US; 1 January 1974 (1974-01-01), CHAMBENOIS, DANIEL ET AL: "Synthesis and stereochemistry of some cyclic acetals", XP002781709, retrieved from STN Database accession no. 1974:449603 & CHAMBENOIS, DANIEL ET AL: "Synthesis and stereochemistry of some cyclic acetals", COMPTES RENDUS DES SEANCES DE L'ACADEMIE DES SCIENCES, SERIE C: SCIENCES CHIMIQUES , 278(20), 1231-4 CODEN: CHDCAQ; ISSN: 0567-6541, 1974,
- DATABASE CA [Online] CHEMICAL ABSTRACTS SERVICE, COLUMBUS, OHIO, US; 1 January 1984 (1984-01-01), SUGAI, SABURO ET AL: "A simple and efficient conversion of aldehyde acetals into esters", XP002781710, retrieved from STN Database accession no. 1984:156319 & SUGAI, SABURO ET AL: "A simple and efficient conversion of aldehyde acetals into esters", CHEMICAL & PHARMACEUTICAL BULLETIN , 32(1), 99-105 CODEN: CPBTAL; ISSN: 0009-2363, 1984, DOI: 10.1248/CPB.32.99 10.1248/CPB.32.99

## Description

The present invention relates to the use of the cyclic acetal of the formula (1.3) as defined below or of stereoisomers or of stereoisomer mixtures thereof as an aroma chemical, to the use thereof for modifying the scent character of a fragranced composition; and to an aroma chemical composition containing said cyclic acetal or a stereoisomer or a mixture of stereoisomers thereof..

### TECHNICAL BACKGROUND

Aroma chemicals, especially fragrances, are of great interest especially in the field of cosmetics and cleaning and laundry compositions. Fragrances of natural origin are mostly expensive, often limited in their available amount and, on account of fluctuations in environmental conditions, are also subject to variations in their content, purity etc. To circumvent these undesirable factors, it is therefore of great interest to create synthetic substances which have organoleptic properties that resemble more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should also have additional positive secondary properties, such as e.g. an efficient preparation method, the possibility of providing better sensory profiles as a result of synergistic effects with other fragrances, a higher stability under certain application conditions, a higher extendability, a better staying power, etc.

However, since even small changes in chemical structure bring about massive changes in the sensory properties such as odor and also taste, the targeted search for substances with certain sensory properties such as a certain odor is extremely difficult. The search for new fragrances and flavorings is therefore in most cases difficult and laborious without knowing whether a substance with the desired odor and/or taste will even actually be found.

EP-A-0278020 relates to 4,4,7,7-tetramethyl-1,3-dioxacycloheptanes which carry in the 2-position an alkyl or cycloalkyl group which may be interrupted by an O atom and their use as fragrance.

JP 2003-113392 relates to a cosmetic composition containing at least one perfume selected from the group consisting of (A) a top note ingredient, (B) a middle note ingredient, (C) a base note ingredient and (D) a natural perfume composition. Middle note ingredients are among many others cyclic acetals, these are however always derived from benzaldehyde.

US 1,837,273 relates to cyclic acetals deriving from araliphatic aldehydes. The compounds are said to have generally a pronounced intensive and very agreeable odor. The compounds have a substitution pattern different from the present compound (I.3).

It was the object of the present invention to provide new aroma chemicals. These should have pleasant organoleptic properties. It was a further object of the present invention to provide substances which can be used as an aroma chemical in ready-to-use compositions. In particular, odor-intensive substances having a pleasant odor are sought. Furthermore, they should be combinable with other aroma chemicals, allowing the creation of novel advantageous sensory profiles. In addition, these aroma chemicals should be obtainable from redily available starting materials, allowing their fast and economic manifacturing, and should be free of toxicological concerns.

This object is achieved by the compound of formula (1.3) as shown below or stereoisomers thereof.

### SUMMARY OF THE INVENTION

The invention relates to the use of a compound of the formula 1.3 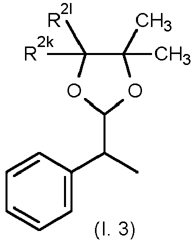 wherein
R^{2k} and R^{2l} are methyl
or of a stereoisomer thereof or of a mixture of stereoisomers thereof,
as an aroma chemical.

Another aspect of the invention is the use of the compound (1.3) or a stereoisomer thereof or a mixture of at least two of the stereoisomers thereof for modifying the scent character of a fragranced composition, e.g. of a fragranced ready-to-use composition.

Yet another aspect of the invention is a composition comprising the compound (1.3) or a stereoisomer thereof or a mixture of at least two of its stereoisomers and one further component selected from the group consisting of one or more other aroma chemicals (i.e. aroma chemicals different from compounds (1.3) and stereoisomers thereof) and a non-aroma chemical carrier, where the non-aroma chemical carrier is in particular selected from the group consisting of surfactants, oil components and solvents.

Described is moreover a method for preparing a fragranced composition, e.g. a fragranced ready-to-use composition, or for modifying the scent character of a fragranced composition, e.g. of a fragranced ready-to-use composition, comprising incorporating the compound of the formula (1.3) or a stereoisomer thereof or a mixture of stereoisomers thereof into said composition.

Described is also a method for preparing the compound (1.3) or a stereoisomer thereof or mixtures of at least two of the stereoisomers thereof; and to a composition obtainable by this method.

The compound of formula (1.3) is known, but not described as aroma chemical.

The compound of formula 1.3 (CAS 124400-18-6) is e.g. known from Journal of Organic Chemistry 1990, 55(3), 1114-1117.

The compound of formula (1.3), its stereoisomers and the mixtures of its stereoisomers possess advantageous organoleptic properties, in particular a pleasant odor. Therefore, they can be favorably used as an aroma chemical for example in perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions, crop protection compositions and other ready-to-use compositions.

By virtue of their physical properties, the compound of formula (1.3), its stereoisomers or the mixtures of its stereoisomers have particularly good, virtually universal solvent properties for other fragrances and other customary ingredients in fragranced ready-to-use compositions such as, in particular, perfume compositions. Therefore, the compound of formula (1.3), its stereoisomers or the mixtures of its stereoisomers are favorably combinable with other aroma chemicals, allowing, in particular, the creation of perfume compositions having novel advantageous sensory profiles.

Furthermore, the compound of formula (1.3), its stereoisomers or the mixtures of its stereoisomers can be produced in good yields and purities by a simple synthesis which generally requires only one step, starting from readily available starting compounds. Thus, the compound of formula (1.3), its stereoisomers or the mixtures of its stereoisomers can be produced in large scales and in a simple and cost-efficient manner.

### DETAILED DESCRIPTION OF THE INVENTION

In the context of the present invention, the term "C₁-C₃-alkyl" refers to a linear or branched alkyl group having 1 to 3 carbon atoms. Examples are methyl, ethyl, n-propyl and isopropyl.

The term "stereoisomers" encompasses optical isomers, such as enantiomers or diastereomers, the latter existing due to more than one stereogenic center in the molecule, as well as geometrical isomers (cis/trans isomers) as a specific form of diastereomers. The compound of the formula (1.3) has a stereogenic center, namely the carbon atom carrying the phenyl ring, the cyclic acetal group and the methyl group. Disclosed are both the pure enantiomers or diastereomers and mixtures thereof. The invention provides the use of the pure enantiomers or of the pure diastereomers of the compound (1.3) or of mixtures thereof.

In the present context, the term "compound (1.3)" or "compound of formula (1.3)", when not defined as a specific stereoisomer or a specific mixture of stereoisomers, refers to the form of the compound as it is obtained in a non-stereoselective method used for its production. The term is however also used if it is not necessary or not possible to specify in more detail the stereochemistry of the compound (1.3).

The compound of formula (1.3), the stereoisomers thereof and the mixtures of stereoisomers thereof are useful as aroma chemicals.

The term "aroma chemical" denotes a substance which is used to obtain a sensory impression, to be more precise an olfactory or flavor impression, in particular a fragrance or flavor impression. The term "olfactory" denotes an odor impression without any positive or negative judgement, while the term "fragrance" (also termed "perfume" or "scent") is connected to an odor impression which is generally felt as pleasant. A flavor induces a taste impression.

"Pleasant odor", "pleasant odor impression", "pleasant odiferous properties" are hedonistic expressions which describe the niceness and conciseness of an odor impression conveyed by an aroma chemical. The more general hedonistic expressions "advantageous sensory properties" or "advantageous organoleptic properties" describe the niceness and conciseness of an organoleptic impression conveyed by an aroma chemical. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

The term "odor-intensive substances" refers to substances or aroma chemicals exhibiting intense odor impressions. Intense odor impressions are to be understood as meaning those properties of aroma chemicals which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

Preferably, the compound of formula (1.3) or stereoisomers thereof or mixtures of stereoisomers thereof as defined above are used as a fragrance.

In particular, the compound 1.3 or a stereoisomer or a mixture of stereoisomers thereof is used to impart a weak, floral, honey note; or is used to produce a scent with a weak, floral, honey note.

The compound (1.3), the stereoisomers thereof or the stereoisomer mixtures thereof are generally used in a ready-to-use composition, in particular in a fragranced ready-to-use composition. "Fragranced ready-to-use composition", as used herein, refers to a ready-to-use composition which predominately induces a pleasant odor impression.

Fragranced ready-to-use compositions are for example compositions used in personal care, in home care, in industrial applications as well as compositions used in other applications, such as pharmaceutical compositions or crop protection compositions.

Preferably, the compounds (1.3), the stereoisomers thereof or the stereoisomer mixtures thereof are used in a composition selected from the group consisting of perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions and crop protection compositions. The compounds (1.3), the stereoisomers thereof or the stereoisomer mixtures thereof are used as an aroma chemical, preferably as a fragrance, in the above compositions.

In particular, the above compound 1.3 or a stereoisomer or a mixture of stereoisomers thereof is used to impart a weak, floral, honey note to the above-listed compositions.

Details to the above-listed compositions are given below.

In addition to the olfactory properties, the compound (1.3), the stereoisomers thereof or the stereoisomer mixtures thereof exhibit advantageous secondary properties.

For example, they can provide better sensory profiles as a result of synergistic effects with other fragrances, which means that they can provide a booster effect for other fragrances. They are therefore suitable as boosters for other fragrances.

Accordingly, another aspect of the invention relates to the use of the compound (1.3), the stereoisomers thereof or the stereoisomer mixtures thereof for modifying the scent character of a fragranced composition; and specifically to the use as a booster for other fragrances.

Booster effect means that the substances enhance and intensify in perfumery formulations the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione^{®} (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compound (1.3), the stereoisomers thereof or the stereoisomer mixtures thereof are generally used in an amount of 0.1 - 20% by weight, preferably in an amount of 0.5 to 5% by weight, in particular in an amount of from 0.6 to 3% by weight, based on the total weight of the fragrance mixture.

Furthermore, the compound (1.3), the stereoisomers thereof or the stereoisomer mixtures thereof can have further positive effects on the composition in which they are used. For example, they can enhance the overall performance of the composition into which they are incorporated, such as the stability, e.g. the formulation stability, the extendability or the staying power of the composition.

In another aspect, the present invention relates to an aroma chemical composition comprising the compound (1.3), the stereoisomers thereof or the stereoisomer mixtures thereof. The term "aroma chemical composition", as used herein, refers to a composition which induces a pleasant odor impression.

Preferably, the aroma chemical composition comprises
- the compound of formula (1.3) or a stereoisomer thereof or a mixture of stereoisomers thereof; and
- at least one further aroma chemical and/or a non-aroma chemical carrier, where the non-aroma chemical carrier is in particular selected from the group consisting of surfactants, oil components and solvents.

The further aroma chemical is of course different from the compound of formula (1.3) or its stereoisomers or mixtures of its stereoisomers.

By virtue of their physical properties, the compound of formula (1.3), its stereoisomers or the mixtures of its stereoisomers have particularly good, virtually universal solvent properties for other fragrances and other customary ingredients in fragranced ready to use compositions such as, in particular, perfume compositions. Therefore, they are well combinable with other aroma chemicals, allowing, in particular, the creation of perfume compositions having novel advantageous sensory profiles. Especially, as already explained above, they can provide a booster effect for other fragrances.

Accordingly, in one preferred embodiment, the aroma chemical composition comprises the compound of formula (1.3) or a stereoisomer thereof or a mixture of stereoisomers thereof as defined above; and at least one further aroma chemical.

The further aroma chemical can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60% by weight) (Hedione⁹, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lilial²), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), oc-tahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide¹), 15-cyclo-pentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol (Florol⁹), 2-ethyl-4-(2,2,3-trimethyl-3-cyclopenten-1-yl)-2-buten-1-ol (Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)propanal (Helional³), 3-(4-ethylphenyl)-2,2-dimethylpropanal (Florazon¹), 7-methyl-2H-1,5-benzodioxepin-3(4H)-one (Calone), 3,3,5-trimethylcyclohexyl acetate (preferably with a content of cis isomers of 70% by weight) or more and 2,5,5-trimethyl-1 ,2,3,4,4a,5,6,7-octahydronaphthalen-2-ol (Ambrinol S¹). Within the context of the present invention, the aforementioned aroma chemical(s) are accordingly preferably combined with the compound of formula (1.3) or a stereoisomer thereof or a mixture of stereoisomers thereof as defined above.

A further embodiment of the invention relates to a composition comprising the compound of formula (1.3) or a stereoisomer thereof or a mixture of stereoisomers thereof as defined above and at least one further aroma chemical selected from the group consisting of methyl benzoate, benzyl acetate, geranyl acetate, 2-isobutyl-4-methyltetrahydro-2H-pyran-4-ol and linalool.

A further embodiment of the invention relates to a composition comprising the compound of formula (1.3) or a stereoisomer thereof or a mixture of stereoisomers thereof as defined above and 2-isobutyl-4-methyltetrahydro-2H-pyran-4-ol.

A further embodiment of the invention relates to a composition comprising the compound of formula (1.3) or a stereoisomer thereof or a mixture of stereoisomers thereof as defined above and methyl benzoate.

Where trade names are given above, these refer to the following sources:
1 trade name of Symrise GmbH, Germany;
2 trade name of Givaudan AG, Switzerland;
3 trade name of International Flavors & Fragrances Inc., USA;
5 trade name of Danisco Seillans S.A., France;
9 trade name of Firmenich S.A., Switzerland;
10 trade name of PFW Aroma Chemicals B.V., the Netherlands.

Further aroma chemicals with which the compound of formula (1.3) or a stereoisomer thereof or a mixture of stereoisomers thereof as defined above can be combined e.g. to give a composition according to the invention can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alphapinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
the aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
the esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
the acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; betaisomethylionone; alpha-irone; alpha-damascone; beta-damascone; betadamascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano-naphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; betasinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5-trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopenta-decenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclo-hexadecen-1-one; 7-cyclohexadecen-1-one; (7/8)-cyclohexadecen-1-one; 9-cyclo-heptadecen-1-one; cyclopentadecanone; cyclohexadecanone;
the cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
the cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1 ,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahy-dro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
the araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
the aromatic and araliphatic aldehydes such as e.g. benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
the aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
the nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropyl-pyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
the heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cisand trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

Advantageous are combinations with aroma chemicals with a sweet note, such as vanillin, 2,5-dimethyl-4-hydroxy-2H-furan-3-one (furaneol) or 3-hydroxy-2-methyl-4H-pyran-4-one (maltol), of the sweet note of which is boosted by the compound (1.3) or its stereoisomers.

A further aspect of the invention is directed to a composition comprising the compound of formula (1.3) or a stereoisomer thereof or a mixture of stereoisomers thereof as defined above and at least one component selected from the group consisting of surfactants, emollients (oil component) and solvents.

One embodiment of the invention is directed to a composition comprising the compound of formula (1.3) or a stereoisomer thereof or a mixture of stereoisomers thereof as defined above and at least one solvent.

In the context of the present invention, a "solvent" serves for the dilution of the compound of formula (1.3) or a stereoisomer thereof or a mixture of stereoisomers thereof as defined above to be used according to the invention without having its own odiferous properties. Some solvents have fixing properties at the same time.

The one or more solvent(s) can be present in the composition from 0.01 to 99% by weight based on the composition. In a preferred embodiment of the invention, the composition comprise 0.1 to 90 weight %, preferably 0.5 to 80 weight% of solvent(s) based on the composition. The amount of solvent(s) can be chosen depending on the composition. In one embodiment of the invention, the composition comprises 0.05 to 10 weight%, preferably 0.1 to 5 weight%, more preferably 0.2 to 3 weight% based on the composition. In one embodiment of the invention, the composition comprises 20 to 70 weight%, preferably 25 to 50 weight% of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compound of formula (1.3), its stereoisomers and its stereoisomer mixtures are suitable for use in surfactant-containing compositions. According to their characteristic scent profiles, they can especially be used for the perfuming of surfactant-containing compositions such as, for example, cleaners (in particular laundry care products and all-purpose cleaners).

One embodiment of the invention is therefore directed to a composition comprising the compound of formula (1.3) or a stereoisomer thereof or a mixture of stereoisomers thereof as defined above and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C₈ to C₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C12- 8 alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart^{®} series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising the compound of formula (1.3) or a stereoisomer thereof or a mixture of stereoisomers thereof as defined above and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15%by weight based on the composition.

The oil components may be selected, for example, from Guerbet alcohols based on fatty alcohols con taining 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl ole- ate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol@ CC), Guerbet carbonates based on fatty alcohols containing 6 to 18 and preferably 8 to 10 carbon atoms, esters of benzoic acid with linear and/or branched C₆ to C₂₂-alcohols (for example Finsolv^{®} TN), linear or branched, symmetrical or nonsymmetrical dialkyl ethers containing 6 to 22 carbon atoms per alkyl group such as, for example, dicaprylyl ether (Cetiol^{®} OE), ring opening products of epoxidized fatty acid esters with polyols and hydrocarbons or mixtures thereof.

The compound of formula (1.3), the stereoisomers thereof and the mixtures of stereoisomers thereof as defined above can be used in a wide range of aroma chemical compositions. The olfactory properties, the substance properties (such as solubility in customary solvents and compatibility with further customary constituents of such compositions), as well as the toxicological acceptability of the compound of formula (1.3), the stereoisomers thereof or the mixtures of stereoisomers thereof underline their particular suitability for the stated use purposes and compositions.

Suitable aroma chemical compositions are for example perfume compositions, body care compositions, hygiene articles, cleaning compositions, textile detergent compositions, foods, food supplements, pharmaceutical compositions and crop protection compositions.

Perfume compositions can be selected from fine fragrances, air fresheners in liquid form, gel-like form or a form applied to a solid carrier, aerosol sprays, scented cleaners, perfume candles and oils, such as lamp oils or oils for massage.

Examples for fine fragrances are perfume extracts, Eau de Parfums, Eau de Toilettes, Eau de Colognes, Eau de Solide and Extrait Parfum.

Body care compositions include cosmetic compositions and products for oral and dental hygiene, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semipermanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara, and products for oral and dental hygiene, such as toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, dishwashing detergents both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, antiinflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, polyols, surfactants, electrolytes, organic solvents or silicone derivatives.

The compound of formula (1.3), the stereoisomers thereof and the mixtures of stereoisomers thereof defined above, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spraydrying an emulsion or dispersion comprising the compound of formula (1.3), its stereoisomer, the mixtures of its stereoisomers as defined above and composition obtainable by the above described method, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compound of formula (1.3), its stereoisomer, the mixtures of its stereoisomers as defined above or the composition obtainable by the above described method with a suitable wax-like substance and by extrusion with subsequent solidification, optionally in a suitable solvent, e.g. isopropanol.

Generally, the total amount of the compound of formula (1.3), the stereoisomers thereof or the mixture of stereoisomers thereof in the aroma chemical compositions according to the present invention is typically adapted to the particular intended use or the intended application and can, thus, vary over a wide range. As a rule, the customary standard commercial amounts for scents are used.

The compositions according to the invention can comprise the compound of formula (1.3), the stereoisomers thereof or the mixture of stereoisomers thereof as defined above in an overall amount of from 0.001 to 99.9% by weight, preferably from 0.01 to 90% by weight, more preferably from 0.05 to 80%, in particular from 0.1 to 60% by weight, more particularly from 0.1 to 40% by weight, e.g. from 0.1 to 10% by weight or 0.1 to 15% by weight, based on the total weight of the composition.

In one embodiment of the invention, the compositions comprise the compound of formula (1.3), the stereoisomers thereof or the mixture of stereoisomers thereof as defined above in an overall amount of from 0.001 to 5 weight%, preferably from 0.01 to 2 weight% based on the total weight of the composition.

Described is moreover a method of preparing an aroma chemical composition, in particular a fragranced composition, especially a fragranced ready-to-use composition, or for modifying the scent character of an aroma chemical composition, in particular of a fragranced composition, especially of a fragranced ready-to-use composition, comprising incorporating the compound of formula (1.3), a stereoisomer thereof or a mixture of stereoisomers thereof as defined above into said composition.

In particular, a method of preparing a perfume composition, body care composition, hygiene article, cleaning composition, textile detergent composition, food, food supplement, pharmaceutical composition or crop protection composition is described, comprising including the compound of formula (1.3), the stereoisomers thereof or the mixture of stereoisomers thereof as defined above or in a perfume composition, body care composition, hygiene article, cleaning composition, textile detergent composition, food, food supplement, pharmaceutical composition or crop protection composition.

In another embodiment, a method can be used for imparting a weak, floral, honey note to a perfume composition, body care composition, hygiene article, cleaning composition, textile detergent composition, food, food supplement, pharmaceutical composition or crop protection composition, which comprises including the compound of formula 1.3 or a stereoisomer or a mixture of stereoisomers thereof as defined above in a perfume composition, body care composition, hygiene article, cleaning composition, textile detergent composition, food, food supplement, pharmaceutical composition or crop protection composition.

The compound of the formula (1.3) can be prepared by the methods as described in the below schemes or in the synthesis descriptions of the working examples, or by standard methods of organic chemistry. In the following schemes, (I) stands for (1.3), R¹ and R² stand for methyl, m stands for 4 and n stands for 0.

To be more precise, the compound (1.3) [depicted in the below schemes as compound (I)] can be prepared by standard methods for preparing cyclic acetals, such as reacting 2-phenyl-propanal with the suitable diol, as shown in scheme 1 below. The reaction of alkanal 1 with diol 2 is generally carried out under acidic conditions, using for example hydrochloric acid, sulfuric acid, acetic acid, trifluoroacetic acid, methanesulfonic acid or p-toluene sulfonic acid or using a polymeric acid, such as a strongly acidic cation exchanger. The term "strongly acidic cationic exchanger" refers to a cationic exchanger in the H⁺ form which has strongly acidic groups. The strongly acidic groups are generally sulfonic acid groups; they are generally bonded to a polymer matrix, which can be e.g. gel-like and/or macroporous. Preference is given to styrene (co)polymers containing sulfonic acid groups, specifically to styrene-divinyl benzene copolymers containing sulfonic acid groups (e.g. a resin of the Amberlyst^{®} brand from Rohm and Haas, such as Amberlyst^{®} 131). Further details are given below in context with schemes 2, 2a or 3. Suitably, the water formed in the reaction is removed, generally by distillation, in order to promote the acetal formation. The reaction is generally carried out in an organic solvent. Suitable solvents are e.g. alkanes, such as pentane or hexane, cycloalkanes, such as cyclohexane, aromatic hydrocarbons, such as toluene and the xylenes, alphatic ethers, such as diethyl ether, diisopropylether or methyl-tert-buty ether, cyclic ethers, such as tetrahydrofuran or the dioxanes, or carboxylic acid esters, such as ethyl acetate.

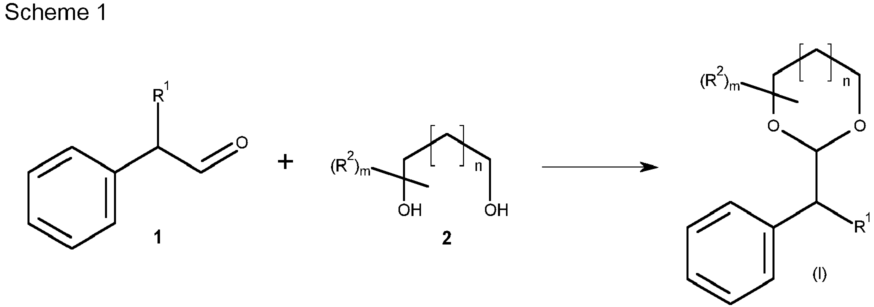

Alternatively, instead of the diol 2, an olefinically unsaturated alkanol 3 can be reacted with aldehyde 1 under aqueous acidic conditions, as shown in scheme 2 It is assumed that the C-C double bond is hydrated to give intermediately a diol, which then reacts with the aldehyde to the acetal compound (I). Suitable acids are those listed above, among which strongly acidic cation exchangers have proved particularly useful. As said above, the term "strongly acidic cationic exchanger" refers to a cationic exchanger in the H⁺ form which has strongly acidic groups. The strongly acidic groups are generally sulfonic acid groups; they are generally bonded to a polymer matrix, which can be e.g. gel-like and/or macroporous. Preference is given to styrene (co)polymers containing sulfonic acid groups, specifically to styrene-divinyl benzene copolymers containing sulfonic acid groups. Commercial examples for such cationic exchangers are Lewatit^{®} (Lanxess), Purolite^{®} (The Purolite Company), Dowex^{®} (Dow Chemical Company), Amberlite^{®} (Rohm and Haas Company), Amberlyst^{®} (Rohm and Haas Company). Preferred strongly acidic cation exchangers are: Lewatit^{®} K 1221, Lewatit^{®}K 1461, Lewatit@ K 2431, Lewatit^{®}K 2620, Lewatit^{®} K 2621, Lewatit^{®}K 2629, Lewatit^{®} K 2649, Amberlite^{®} FPC 22, Amberlite^{®} FPC 23, Amberlite^{®} IR 120, Amberlyst^{®} 131, Amberlyst^{®} 15, Amberlyst^{®} 31, Amberlyst^{®} 35, Amberlyst^{®} 36, Amberlyst^{®} 39, Amberlyst^{®} 46, Amberlyst^{®} 70, Purolite^{®} SGC650, Purolite^{®} C100H, Purolite^{®} C150H, Dowex^{®} 50X8, Serdolit^{®} red and Nation^{®} NR-50. Specifically, resins of the Amberlyst^{®} brand from Rohm and Haas, and very specifically Amberlyst^{®} 131 is used. Alternatively, the cation exchanger can be a perfluorinated ion exchange resin, sold e.g. under the Nafion^{®} brand of DuPont.

Alkanol 3 and aldehyde 1 are preferably reacted in the presence of a strongly acidic cation exchanger and also in the presence of water. Water can be added additionally to the reaction mixture, but can also be just added in form of the water that is present in the strongly acidic cation exchanger if this is used in wet form. The reaction is preferably carried out in the presence of about at least 10 mol % of water, the amount of water referring to 1 mol of 3 or 1, whichever is used in deficit amounts (or, if 3 and 1 are used in equimolar amounts, to 1 mol of one of 3 or 1), and in particular in the presence of at least equimolar amounts of water, the amount of water referring again to 1 mol of 3 or 1, whichever is used in deficit amounts or, if 3 and 1 are used in equimolar amounts, to 1 mol of one of 3 or 1. Starting compounds 1 and 3 are usually applied in a molar ratio of 2:1 to 1:2, preferably in a ratio of 1.5:1 to 1:1.5, in particular of 1.2:1 to 1:1.2 and specifically approximatly in an equimolar ratio. "Approximately" includes a deviation of ± 10%, preferably ± 5% from the exact equimolar ratio and takes account of weighing errors or errors due to incomplete transfer of the weighed compound to the reaction vessel and the like. The amount of strongly acidic cation exchanger is not critical and can be chosen freely within wide limits taking into consideration the economic and processing aspect. Accordingly, the reaction can be carried out either in the presence of catalytic amounts or in the presence of large excesses of the strongly acidic cation exchanger. Usually, the strongly acidic cation exchanger is used in an amount of from about 5 up to about 40% by weight, preferably in an amount of from about 10 to about 40% by weight and particularly preferably in an amount of from about 15 to about 30% by weight, in each case based on the sum of the weights of 1 and 3. Here, the figures refer to the ready-to-use cation exchanger which is generally pretreated with water and accordingly can comprise amounts of up to about 70% by weight, preferably of about 30 to about 70% by weight and particularly preferably of about 40 to about 70% by weight of water. Particularly in the case of a discontinuous procedure, an additional addition of water when carrying out the process may therefore be superfluous. The specified strongly acidic cation exchangers can be used either individually or else in the form of mixtures. The reaction in the presence of a strongly acidic cation exchanger can, if desired, also additionally be carried out in the presence of a solvent that is inert under the reaction conditions. Suitable solvents are those listed above in context with scheme 1. The specified solvents can be used on their own or in the form of mixtures with one another. Preferably, the reaction is carried out in the presence of a strongly acidic cation exchanger without the addition of an organic solvent. The reactants can in principle be contacted with one another in any desired sequence. For example, 3 and the cationic exchange resin, optionally dissolved or dispersed in an inert solvent, can be initially charged and mixed with each other. The obtained mixture can then be admixed with 1. Conversely, 1, optionally dissolved or dispersed in an inert solvent, can be initially charged and admixed with a mixture of 3 and the cationic exchange resin. Alternatively, 1 may first be mixed with the cationic exchange resin and the mixture is then admixed with 3. As a further alternative all reactants can be added simultaneously to the reaction vessel. It has however been found to be beneficial to initially charge the reaction vessel with a mixture of 3 and the cationic exchange resin, possibly as a dispersion or solution in a solvent, but preferably without solvent, and then to add 1, which is employed in dissolved form or, preferably, as such. The reaction is generally performed at a temperature in the range from 10 to 150°C, preferably in the range from 30 to 100°C, more preferably in the range from 40 to 90°C and specifically in the range from 50 to 80°C. The reaction pressure is not critical, so that generally the reaction is carried out at ambient pressure.

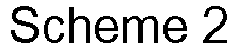

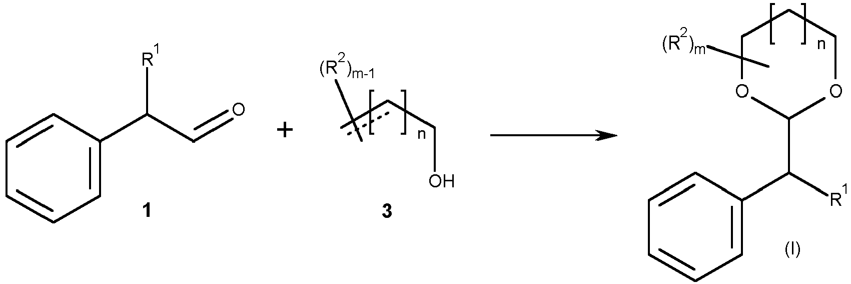

The dotted line in compound 3 indicates that one (and only one) of the bonds is a C-C double bond. The index of R² in compound 3 is (m-1) because hydration of the double bond generally takes place in a Markovnikov manner. This means that even if the double bond in 3 is in a terminal position (a-olefin; CH₂=CH-...), the hydration will result in the OH group being bound to the β-carbon atom of the double bond (=CH). This means in turn that the CH₃ group formed by the addition of H⁺ to the α-carbon atom of the double bond (CH₂=) will be a radical R² on the acetal (I); therefore, the index of R² in 3 is (m-1).

The reaction mixtures are worked up in a customary manner, for example by filtrating off the acidic catalyst, if this was a resin, mixing with water, neutralizing, separating the phases, isolating the product from the organic phases and, if appropriate, purifying the crude products by usual methods, e.g. by distillative, extractive or chromatographic methods. The above reactions can result in a mixture of various isomers, such as optical isomers. If desired, these can be separated from each other by customary means, but generally this is not necessary, at least not if a reaction according to scheme 1 is applied. In the case of the reaction depicted in scheme 2, however, more complex reaction products are often obtained which can contain inter alia the reaction product of the possibly competing Prins reaction in which two moles of aldehyde 1 react with one mole of alkanol, or saturated or unsaturated rings containing just one oxygen atom (cf. US 2014/0107352). In this case, it is expedient to isolate the desired product (I) from the reaction mixture, e.g. via distillative, extractive or chromatographic methods.

The invention is illustrated by the following examples.

### EXAMPLES

### 1. Preparation examples

### Analytics:

The purity of the products was determined by Gas Chromatography area-%:
GC-system: Agilent 6890 N
GC-Column: HP5 (30 m (Length), 0.25 mm (ID), 0.32 micrometer (film)), flow 1mL/min;
Temperature program: 60 °C to 250 °C at 6°/min, 250°C for 10min.

The products were identified by ¹³C NMR.

### 1.1 Synthesis of 4,4-dimethyl-2-(1-phenylethyl)-1,3-dioxane (not according to the invention)

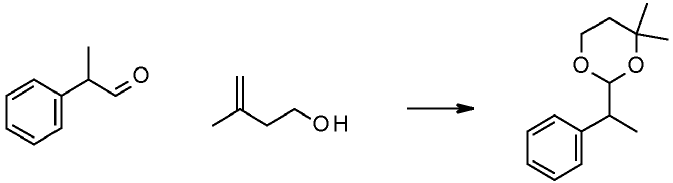

Isoprenol (3-methylbut-3-en-1-ol) (60 g, 0.69 mol), together with Amberlyst^{®} 131 resin (30 g; wet, strongly acidic cation exchanger resin from Rohm&Hass; washed with water, then with methanol and again with water before use) was introduced into a 250mL HWS reactor (from Hans W. Schmidt GmbH & Co. KG, Maiz, Germany). Stirring of the mixture was started. 2-Phenylpropionaldehyde (90.7 g, 0.68 mol) was then added dropwise at room temperature (lightly exotherm). The reaction mixture was warmed up to 70°C and stirred for 5h. After cooling overnight, the resin was filtered. To the filtrate 100mL of ethyl acetate and 100mL of water were added. After phase separation, the organic phase was washed with 100mL of a saturated solution of NaHCO₃ and with 100mL of water. The organic phase was dried with sodium sulfate and the solvent was removed in the rotatory evaporator to give 134.3g of crude product that contained 65% of the desired title compound and 12% of unreacted 2-phenylpropionaldehyde [according to GC (area %)]. Ca 20% was identified as the Prins condensation product. 40g of the crude product were purified by distillation at reduced pressure. The product was isolated as a clear liquid with a purity >97%. The NMR indicated the presence of the product as a 1:1 diastereomer mixture (two pairs of enantiomers at two stereogenic centers (C4 and C9)).

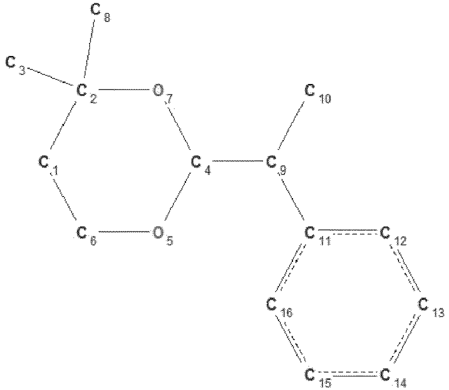

| Atom number | ¹³C-NMR (125 MHz; CDCI3) δ (multiplicity) |
|---|---|
| C1 | 35,82/ 35,80 (t) |
| C2 | 71,23/ 71,26 (s) |
| C3 | 21,49/ 21,61 (q) |
| C4 | 97,85/ 98,30 (d) |
| C6 | 63,06/ 63,19 (t) |
| C8 | 31,65/ 31,70 (q) |
| C9 | 44,65/ 44,91 (d) |
| C10 | 15,51/ 16,64 (q) |
| C11 | 143,31 (s) |
| C12/ C16 | 128,28/ 128,08 (d) |
| C13/ C15 | 127,95/ 128,05 (d) |
| C14 | 126,13 (d) |

### 1.6 Synthesis of 4,4,5,5-tetramethyl-2-(1-phenylethyl)-1,3-dioxane

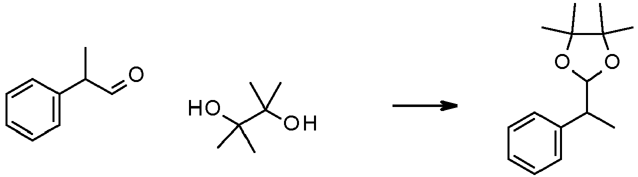

2-Phenylpropionaldehyde (15 g, 0.112 mol), 2,3-dimethyl-2,3-butanediol (17.525 g, 0.145 mol), p-toluenesulfonic acid (425 mg, 0.002 mol) and toluene (100 mL) were mixed in a 250 mL flask that had been connected to a Dean Stark apparatus. The mixture was refluxed for 1.5 h while water was being distilled off. The reaction mixture was then cooled and washed with a saturated solution of NaHCO₃. After phase separation the organic phase was washed with water. The organic phase was dried with sodium sulfate and the solvent was removed in the rotatory evaporator to give 26.1 g of crude product that contained the 99% of the desired title compound. The crude product was purified by distillation at reduced pressure. The product was isolated with a purity of >99%.

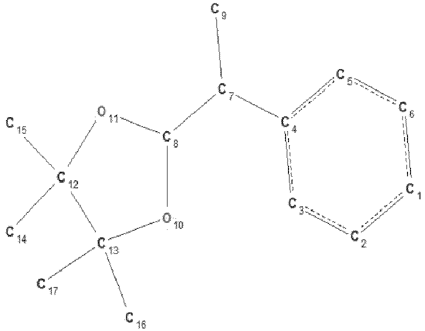

| Atom number | ¹³C-NMR (125 MHz; CDCI3) δ (multiplicity) |
|---|---|
| C1 | 126,39 (d) |
| C2/C6 | 127,98 (d) |
| C3/C5 | 128,55 (d) |
| C4 | 142,43 (s) |
| C7 | 45,06 (d) |
| C8 | 103,64 (d) |
| C9 | 16,25 (q) |
| C12/C13 | 81,76/ 81,69 (s) |
| C14/C15/C16/C17 | 24,13/ 24,06/ 22,25/ 22,16 (q) |

The compound of example 1.1 can be used as the essentially pure R,R-isomer or as the essentially pure S,S-isomer or as the essentially pure R,S-isomer, or as the essentially pure S,R-isomer or as a mixture of at least two of these isomers; e.g. a mixture of the R,R- and the S,S-isomer, e.g. a 50:50 mixture; or a mixture of the R,S- and the S,R-isomer; e.g. a 50:50 mixture, or a mixture of all 4 isomers, e.g. a 25:25:25:25 mixture.

The compound of example 1.6 can be used as the essentially pure R-enantiomer or as the essentially pure S-enantiomer or as a mixture of the two enantiomers; e.g. a 50:50 mixture (racemate); the stereogenic center being the C atom carrying the phenyl ring, the methyl group and the dioxane ring.

### 2. Olfactory assessment

In order to test the quality and intensity of the odor of the compound obtained in the preparation examples, scent strip tests were performed.

For this purpose, strips of absorbent paper were dipped into a solution containing 1 to 10 % by weight solution of the compound to be tested in ethanol. After evaporation of the solvent (about 30 sec.) the scent impression was olfactively evaluated by a trained perfumer.

### Results:

**Table 1: Results of the scent tests**

| Example no. | Compound | Odor Description |
|---|---|---|
| 1.1 | 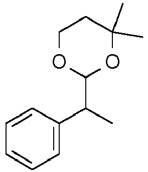 | Floral, dandelion, acacia, natural, honey |
| 1.6 | 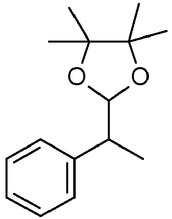 | Weak, floral, honey |

### Advantageous perfume components

The compounds obtained in the preparation examples, to be more precise in examples 1.1 and 1.6 were formulated in the perfume compositions according to tables 3 and 4.

**Table 3: Perfume oil compositions 1A and 1B**

| | **1A** | **1B** |
|---|---|---|
| Benzoe Siam 20% | 711 | 711 |
| Rosewood Oil brasilian | 85 | 85 |
| Copaivabalm rect. | 9 | 9 |
| Linalyl-benzoate | 31 | 31 |
| 3-cis-Hexenyl-salicylate | 21 | 21 |
| Geranyl-acetate | 47 | 47 |
| Ethyl-benzoate | 12 | 12 |
| Cinnamyl-acetate | 2 | 2 |
| Benzyl-acetate | 71 | 71 |
| Methyl-anthranilate 10% | 5 | 5 |
| Bayoil St. Thomas 10% | 5 | 5 |
| Compound of example 1.1 | 0 | 20 |
| | 1000 | 1020 |

**Table 4: Perfume oil compositions 2A and 2B**

| | **2A** | **2B** |
|---|---|---|
| Ethyl Caproate | 1 | 1 |
| Ethyl Acetate | 1 | 1 |
| Iso Amyl Butyrate | 1 | 1 |
| Maltol or Veltol | 1 | 1 |
| Geranyl Butyrate | 2 | 2 |
| Ethyl Vanilline 10% DPG | 2 | 2 |
| Cis 3 Hexenyl Acetate | 3 | 3 |
| Allyl Caproate | 3 | 3 |
| Verdural B 10% DPG | 3 | 3 |
| Oxyphenylon | 3 | 3 |
| Hexyl Butyrate | 4 | 4 |
| Ethyl Decadienoate 10% DPG | 4 | 4 |
| DM.B.C. Butyrate | 4 | 4 |
| Ethyl Maltol or Veltol Plus | 4 | 4 |
| Cyclaprop | 5 | 5 |
| Iso Amyl Acetate | 5 | 5 |
| Cis 3 Hexenol 10% DPG | 6 | 6 |
| D.M.B.C. Acetate | 7 | 7 |
| Aldehyde C 16 100% | 8 | 8 |
| Geranyl Propionate | 8 | 8 |
| Ethyl 2 Methyl Butyrate | 8 | 8 |
| Decalactone Gamma | 10 | 10 |
| Orange Bresil Oil | 10 | 10 |
| Ethyl Aceto Acetate | 10 | 10 |
| Linalool | 15 | 15 |
| Benzyl Acetate | 15 | 15 |
| Aldehyde C 14 100% | 20 | 20 |
| Citronellol | 25 | 25 |
| Linalyl Acetate | 30 | 30 |
| Geranyl Acetate | 35 | 35 |
| Vertenex | 45 | 45 |
| Citronellyl Acetate | 50 | 50 |
| Verdox | 54 | 54 |
| Galaxolide 50 DEP | 100 | 100 |
| Hexyl Acetate | 190 | 190 |
| Mono Propylene Glycol | 300 | 300 |
| Compound of example 1.1 | 0 | 200 |
| | 1000 | 1200 |

Perfume oil composition 7 corresponds to perfume oil composition 1B, where the compound of example 1.1 is replaced by the same amount of the compound of example 1.6.

Perfume oil composition 14 corresponds to perfume oil composition 2B, where the compound of example 1.1 is replaced by the same amount of the compound of example 1.6.

The compound of formula (1.3), a stereoisomer thereof or a mixture of two or more stereoisomers thereof, in particular the product obtained in example 1.6 may be used in a broad range of fragrance applications, e.g. in any field of fine and functional perfumery, such as perfumes, household products, laundry products, body care products and cosmetics. The compound of formula (1.3), a stereoisomer thereof or a mixture of two or more stereoisomers thereof, in particular the product obtained in example 1.6 can be employed in widely varying amounts, depending upon the specific application and on the nature and quantity of other odorant ingredients. The proportion is typically from 0.001 to 20 weight per cent of the application. In one embodiment, the compound of formula (1.3), a stereoisomer thereof or a mixture of two or more stereoisomers thereof, in particular the product obtained in example 1.6 are employed in a fabric softener in an amount of from 0.001 to 0.05 weight per cent. In another embodiment, the compound of formula (1.3), a stereoisomer thereof or a mixture of two or more stereoisomers thereof, in particular the product obtained in example 1.6 are used in fine perfumery in amounts of from 0.1 to 20 weight per cent, more preferably between 0.1 and 5 weight per cent. However, these values are given only by way of example, since the experienced perfumer may also achieve effects or may create novel accords with lower or higher concentrations.

The compound of formula (1.3), a stereoisomer thereof or a mixture of two or more stereoisomers thereof, in particular the product obtained in example 1.6 may be employed into the fragrance application simply by directly mixing the fragrance composition with the fragrance application, or they may, in an earlier step be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of the compound of formula (1.3), a stereoisomer thereof or a mixture of two or more stereoisomers thereof, in particular the product obtained in example 1.6 as a fragrance ingredient, either by directly admixing the compound of formula (1.3), a stereoisomer thereof or a mixture of two or more stereoisomers thereof or a double bond isomer thereof, in particular the product obtained in example 1.6 to the application or by admixing a fragrance composition comprising the compound of formula (1.3), a stereoisomer thereof or a mixture of two or more stereoisomers thereof or a double bond isomer thereof, in particular the product obtained in example 1.6, which may then be mixed to a fragrance application, using conventional techniques and methods.

As used herein, "fragrance application" means any product, such as fine perfumery, e.g. perfume and Eau de Toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odorant. This list of products is given by way of illustration and is not to be regarded as being in any way limiting.

The compound of formula (1.3), a stereoisomer thereof or a mixture of two or more stereoisomers thereof or a double bond isomer thereof, in particular the product obtained in example 1.6 may be used as part of the perfume in the above mentioned applications. The compound of formula (1.3), a stereoisomer thereof or a mixture of two or more stereoisomers thereof or a double bond isomer thereof, in particular the product obtained in example 1.6 may be used alone or as part of a perfume. The term "perfume" is used synonymously to "perfume oil" or "perfume (oil) composition".

In the following tables all amounts are given in weight-% (% b.w.). Conc. means concentration.

**Table 5: Deo pump spray 1**

| **Deo Pump Spray; PIT** | | |
|---|---|---|
| **Component** | **INCI** | **amount %** |
| EMULGADE^{®} SE-PF | Glyceryl Stearate (and) Ceteareth-20 (and) Ceteareth-12 (and) Cetearyl Alcohol (and) Cetyl Palmitate | 3.9 |
| EUMULGIN^{®} B 2 | Ceteareth-20 | 1.6 |
| CETIOL^{®} OE | Dicaprylyl Ether | 5 |
| CETIOL^{®} PGL | Hexyldecanol (and) Hexyldecyl Laurate | 2 |
| Irgasan DP 300 | Triclosan | 0.25 |
| HYDAGEN^{®} DEO | Triethyl Citrate (and) BHT | 2.5 |
| Water, de ionized | Aqua | 19 |
| Eumulgin^{®} HRE 60 | PEG-60 Hydrogenated Castor Oil | 1.5 |
| Perfume oil composition 1B | Perfume | 0.5 |
| Water, de ionized | Aqua | ad 100 |
| | | |
| Preservative | | q.s. |
| Viscosity mPas | < 100 | |

Deo pump spray 7 corresponds to deo pump spray 1, where the perfume oil composition 1B is replaced by the same amount of the perfume oil composition 7.

Deo pump spray 14 corresponds to deo pump spray 1, where the perfume oil composition 1B is replaced by the same amount of the perfume oil composition 14.

**Table 6: Deo pump spray 17**

| Deo Pump Spray | | |
|---|---|---|
| Component | INCI | amount % |
| Eumulgin^{®} HRE 60 | PEG-60 Hydrogenated Castor Oil | 2 |
| Farnesol | Deo ingredient | 0.2 |
| HYDAGEN^{®} DCMF | Chitosan | 0.1 |
| glycolic acid (Fa. Merck) | glycolic acid | 0.04 |
| Water, deionized | | ad 100 |
| Perfume oil composition 1B | Perfume | 0.5 |
| pH value | 4 | |

Deo pump spray 23 corresponds to deo pump spray 17, where the perfume oil composition 1B is replaced by the same amount of the perfume oil composition 7.

Deo pump spray 30 corresponds to deo pump spray 17, where the perfume oil composition 1B is replaced by the same amount of the perfume oil composition 14.

**Table 7: Clean hair conditioner 1**

| **Clear Hair Conditioner** | | |
|---|---|---|
| **Component** | **INCI** | **amount %** |
| DEHYQUART^{®} L 80 | Dicocoylethyl Hydroxyethylmonium Methosulfate (and) Propylene Glycol | 1.0 |
| Propylene Glycol | solvent | 10.0 |
| Eumulgin^{®} HRE 60 | PEG-60 Hydrogenated Castor Oil | 1.0 |
| LAMESOFT^{®} PO 65 | Coco-Glucoside (and) Glyceryl Oleate | 1.0 |
| GLUADIN^{®} W 40 | Hydrolyzed Wheat Gluten Hydrolyzed Wheat Protein | 0.5 |
| Perfume oil composition 1B | Perfume | 0.02 |
| Preservative | | q.s |
| HYDAGEN^{®} HCMF | Chitosan | 10.0 (sol.1%) |
| Water, deionized | | up to 100.0 |
| | | |
| pH-value | 3.5 - 4.0 | |

Clean hair conditioner 7 corresponds to clean hair conditioner 1, where the perfume oil composition 1B is replaced by the same amount of the perfume oil composition 7. Clean hair conditioner 14 corresponds to clean hair conditioner 1, where the perfume oil composition 1B is replaced by the same amount of the perfume oil composition 14.

**Table 8: Face wash gel 1**

| **Face Wash Gel** | | |
|---|---|---|
| **Component** | **INCI** | **amount %** |
| PLANTAPON^{®} 611 L | Sodium Laureth Sulfate (and) Lauryl Glucoside (and) Cocamidopropyl Betaine | 20 |
| PLANTAPON^{®} ACG 35 | Disodium Cocoyl Glutamate | 1 |
| LAMESOFT^{®} PO 65 | Coco-Glucoside (and) Glyceryl Oleate | 2 |
| NaCl | Sodium Chloride | 1.7 |
| PLANTACARE^{®} PS 10 | Sodium Laureth Sulfate (and) Lauryl Polyglucose | 0.3 |
| Perfume oil composition 1B | Perfume | 0.1 |
| Water | Aqua | QS |
| Preservative | | QS |
| Dye | | QS |
| pH 6 to 7 | | |

Face wash gel 7 corresponds to face wash gel 1, where the perfume oil composition 1B is replaced by the same amount of the perfume oil composition 7.

Face wash gel 14 corresponds to face wash gel 1, where the perfume oil composition 1B is replaced by the same amount of the perfume oil composition 14.

**Table 9: Foam bath concentrate 1**

| **Foam Bath Concentrate** | | |
|---|---|---|
| **Component** | **INCI** | **amount %** |
| TEXAPON^{®} K 14 S 70spec. | Sodium Myreth Sulfate | 25 |
| PLANTACARE^{®} 2000 UP | Decyl Glucoside | 20 |
| DEHYTON^{®} K | Cocamidopropyl Betaine | 20 |
| GLUADIN^{®} WP | Hydrolyzed Wheat Gluten Hydrolyzed Wheat Protein | 1 |
| PLANTACARE^{®} PS 10 | Sodium Laureth Sulfate (and) Lauryl Polyglucose | 5 |
| Water, deionized | | ad 100 |
| Citric Acid, 50 % | | 0.5 |
| Perfume oil composition 1B | perfume | 2.0 |
| pH value | 5.5 | |

Foam bath concentrate 7 corresponds to foam bath concentrate 1, where the perfume oil composition 1B is replaced by the same amount of the perfume oil composition 7. Foam bath concentrate 14 corresponds to foam bath concentrate 1, where the perfume oil composition 1B is replaced by the same amount of the perfume oil composition 14.

**Table 10: Hair gel 1**

| **Hair Gel** | | |
|---|---|---|
| **Component** | **INCI** | **amount %** |
| HYDAGEN^{®} HCMF | Chitosan | 0.5 |
| Glycolic Acid (Fa. Merck) | Solvent | 0.2 |
| Water | | ad 100 |
| Jaguar HP 105 (2%swelling) | Thickener | 65 |
| Eumulgin^{®} HRE 60 | PEG-60 Hydrogenated Castor Oil | 1.5 |
| Perfume oil composition 1B | Perfume | 0.1 |
| Ethanol | Evaporation agent | 7 |
| pH-value | 5.8 | |
| Viscosity (mPas), Brook.RVF, 23°C, sp.7, 10rpm = 20,000 | | |

Hair gel 7 corresponds to hair gel 1, where the perfume oil composition 1B is replaced by the same amount of the perfume oil composition 7.

Hair gel 14 corresponds to hair gel 1, where the perfume oil composition 1B is replaced by the same amount of the perfume oil composition 14.

**Table 11: Self foaming bodywash 1**

| **Self foaming bodywash** | | |
|---|---|---|
| **Component** | **INCI** | **amount %** |
| TEXAPON^{®} SB 3 KC | Disodium Laureth Sulfosuccinate | 16.5 |
| DEHYTON^{®} K | Cocamidopropyl Betaine | 6.5 |
| PLANTACARE^{®} 818 UP | Coco Glucoside | 7.5 |
| TEXAPON^{®} NSO | Sodium laureth sulfate | 14.2 |
| LAMESOFT^{®} PO 65 | Coco-Glucoside (and) Glyceryl Oleate | 5 |
| Dow Corning 193 | Dimethicole Copolyol | 1 |
| PLANTACARE^{®} PS 10 | Sodium Laureth Sulfate (and) Lauryl Polyglucose | 0.5 |
| Perfume oil composition 1B | Perfume | 0.5 |
| Kathon CG | Methylchloroisothiazolinone (and) Methylisothiazolinone | 0.05 |
| Water, de-ionized | Aqua | ad 100 |
| Hexylen Glycol (Elf Atochem) | Humectant | 3 |
| UCARE Polymer JR 400 | Polyquaternium-10 | 0.5 |
| pH: 5.5 | | |
| Viscosity: > 100 mPas | | |

Self foaming bodywash 7 corresponds to self foaming bodywash 1, where the perfume oil composition 1B is replaced by the same amount of the perfume oil composition 7. Self foaming bodywash 14 corresponds to self foaming bodywash 1, where the perfume oil composition 1B is replaced by the same amount of the perfume oil composition 14.

**Table 12: Sprayable sun care emulsion 1**

| **Sprayable Sun Care Emulsion** | | |
|---|---|---|
| **Component** | **INCI** | **amount %** |
| EMULGADE^{®} SE-PF | Glyceryl Stearate (and) Ceteareth-20 (and) Ceteareth-12 (and) Cetearyl Alcohol (and) Cetyl Palmitate | 7.8 |
| EUMULGIN^{®} B 3 | Ceteareth-30 | 4.2 |
| CETIOL^{®} CC | Dicaprylyl Carbonate | 5.0 |
| CETIOL^{®} OE | Dicaprylyl Ether | 5.0 |
| Neo Heliopan BB (Haarmann&Reimer) | | 4.5 |
| Neo Heliopan AV (Haarmann&Reimer) | | 7.5 |
| Neo Heliopan 357 (Haarmann&Reimer) | | 2.0 |
| Water, de-ionized | | ad 100 |
| Perfume oil composition 1B | Perfume | 0.05 |
| Eumulgin^{®} HRE 60 | PEG-60 Hydrogenated Castor Oil | 1.2 |
| pH | 5.5 | |
| Viscosity (mPas), RVF spindle 2, 20 °C, 50 rpm < 100 | | |

Sprayable sun care emulsion 7 corresponds to sprayable sun care emulsion 1, where the perfume oil composition 1B is replaced by the same amount of the perfume oil composition 7.

Sprayable sun care emulsion 14 corresponds to sprayable sun care emulsion 1, where the perfume oil composition 1B is replaced by the same amount of the perfume oil composition 14.

**Table 13: Sprayable sun protection emulsion 1**

| **Sprayable Sun Protection Emulsion** | | |
|---|---|---|
| **Component** | **INCI** | **amount %** |
| EMULGADE^{®} SE-PF | Glyceryl Stearate (and) Ceteareth-20 (and) Ceteareth-12 (and) Cetearyl Alcohol (and) Cetyl Palmitate Ceteareth-33 | 9.0 |
| Eumulgin B3 | | 6.0 |
| CETIOL^{®} CC | Dicaprylyl Carbonate | 4.0 |
| Eusolex HMS | Homosalate | 7.0 |
| Parsol MCX (Givaudan Roure) | Ethylhexyl Methoxycinnamate | 7.5 |
| Parsol 1789 | Butyl Methoxydibenzoylmethane | 3.0 |
| Neo Heliopan OS | Ethylhexyl Salicylate | 5.0 |
| Preservative | | q.s. |
| Water, de-ionized | | ad 100 |
| Perfume oil composition 1B | Perfume | 0.2 |
| Eumulgin^{®} HRE 60 | PEG-60 Hydrogenated Castor Oil | 1.2 |
| pH-value | 6.2 | |

Sprayable sun protection emulsion 7 corresponds to sprayable sun protection emulsion 1, where the perfume oil composition 1B is replaced by the same amount of the perfume oil composition 7.

Sprayable sun protection emulsion 14 corresponds to sprayable sun protection emulsion 1, where the perfume oil composition 1B is replaced by the same amount of the perfume oil composition 14.

**Table 14: Emollient facial gel 1**

| **Emollient facial Gel** | | |
|---|---|---|
| **Component** | **INCI** | **amount %** |
| Water | | Ad 100 |
| HISPAGEL^{®} 200 | Glycerin (and) Glyceryl Polyacrylate | 10 |
| CET-OE-Primasponge^{®}BLUE | | 0.5 |
| PLANTACARE^{®} PS 10 | Sodium Laureth Sulfate (and) Lauryl Polyglucose | 0.6 |
| Perfume oil composition 1B | | 0.1 |

Emollient facial gel 7 corresponds to emollient facial gel 1, where the perfume oil composition 1B is replaced by the same amount of the perfume oil composition 7. Emollient facial gel 14 corresponds to emollient facial gel 1, where the perfume oil composition 1B is replaced by the same amount of the perfume oil composition 14.

**Table 15: 2-phases oil foam bath 1**

| **2-Phases-Oilfoambath** | | |
|---|---|---|
| **Component** | **INCI** | **amount % b.w.** |
| PLANTACARE^{®} PS 10 | Sodium Laureth Sulfate (and) Lauryl Polyglucose | 10 |
| Paraffin oil | (low viscosity) | 20 |
| TEXAPON^{®} N 70 | Sodium Laureth sulfate | 13 |
| DEHYTON^{®} PK 45 | Cocamidopropyl Betaine | 8 |
| Perfume oil composition 1B | Perfume | 2 |
| Glycerin (86%) | | 5 |
| Preservative | | q.s. |
| Water, de-ionized | | ad 100.0 |
| pH-value | 5.5 | |

2-phases oil foam bath 7 corresponds to 2-phases oil foam bath 1, where the perfume oil composition 1B is replaced by the same amount of the perfume oil composition 7.

2-phases oil foam bath 14 corresponds to 2-phases oil foam bath 1, where the perfume oil composition 1B is replaced by the same amount of the perfume oil composition 14.

**Table 16: Shampoos 1, 2, 3 and 4**

| **Shampoo** | | | **1** | **2** | **3** | **4** |
|---|---|---|---|---|---|---|
| **Component** | **INCI** | **Conc.** | **% b.w.** | | | |
| Texapon N70 | Sodium Laureth Sulfate | 70% | 12.9 | 12.9 | 14.3 | 14.3 |
| Dehyton PK45 | Cocam idopropyl Betaine | 44 - 46% | 5.4 | 5.4 | 5.4 | 5.4 |
| Plantacare 818UP | Coco-Glucoside | 51 - 53% | 2.0 | 2.0 | - | - |
| PLANTACARE^{®} PS 10 | Sodium Laureth Sulfate (and) Lauryl Polyglucose | 33% | 6.0 | 18.0 | 21.0 | 30.0 |
| Copherol 1250 C | Tocopheryl Acetate | 100% | 1.0 | - | - | - |
| Cetiol J 600 | Oleyl Erucate | 100% | - | 0.7 | - | 1.0 |
| Almond Oil | Prunus Amygdalus Dulcis (Sweet Almond) Oil | 100% | - | - | 0.3 | - |
| Jaguar C162 | Hydroxypropyl Guar Hydroxypropyltrimonium Chloride | 100% | 0.2 | 0.2 | - | - |
| Jaguar Excel | Guar Hydroxypropyltrimonium Chloride | 100% | - | - | 0.25 | 0.25 |
| Arlypon TT | PEG/PPG-120/10 Trimethylolpropane Trioleate (and) | 40 - 50% | | | | |
| | Laureth-2 | 40 - 50% | 1.0 | 2.0 | 1.0 | 2.0 |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 70.7 | 49.0 | 56.95 | 46.2 5 |
| pH value | | | 5.0 | 4.9 | 5.2 | 5.1 |
| Viscosity [mPas] | | | 5600 | 3100 | 4600 | 250 0 |

**Table 17: Shampoos 5, 6, 7 and 8**

| **Shampoo** | | | | **5** | **6** | **7** | **8** |
|---|---|---|---|---|---|---|---|
| **Component** | **INCI** | | **Conc.** | **% b.w.** | | | |
| Sulfopon 1216 G | Sodium Coco-Sulfate | | 90 - 95% | --- | --- | 12.8 | --- |
| Texapon ALS Benz | Ammonium Lauryl Sulfate | | 27 - 28% | --- | --- | --- | 32.0 |
| Plantacare 1200UP | Lauryl Glucoside | | 51 - 53% | 15.0 | --- | --- | --- |
| Plantacare 2000UP | Decyl Glucoside | | 51 - 55% | --- | --- | --- | 6.0 |
| Plantacare 818UP | Coco-Glucoside | | 51 - 53% | --- | 30.0 | 20.4 | --- |
| Plantacare 810UP | Caprylyl/Capryl Glucoside | | 62 - 65% | 12.0 | --- | --- | --- |
| Jaguar Excel | Guar Hydroxypropyltrimonium Chloride | | 100% | 0.2 | 0.25 | 0.2 | 0.3 |
| Dehyton PK45 | Cocam idopropyl Betaine | | 44 - 46% | | | | 11.0 |
| Lamesoft PO65 | Coco-Glucoside (and) Glyceryl Oleate water | 15 - 25 | }28,5 - 34% | 2.0 | 2.0 | 2.0 | 3.0 |
| | | 10 - 20 | | | | | |
| | | 62 - 67 | | | | | |
| Euperlan Green | Lauryl Glucoside (and) Stearyl Citrate | 15 - 25 | }38-44 % | --- | --- | --- | 2.0 |
| | | 15 - 25 | | | | | |
| | | 55 - 65 | | | | | |
| | water | | | | | | |
| Gluadin WLM Benz | Hydrolyzed Wheat Protein | | 21 - 24% | --- | 0.5 | 1.0 | --- |
| PLANTACARE ^{®} PS 10 | Sodium Laureth Sulfate (and) Lauryl Polyglucose | | 33% | 6.0 | 15.0 | 7.5 | 10.5 |
| Copherol 1250 C | Tocopheryl Acetate | | 100% | 0.25 | --- | --- | --- |
| Cetiol J 600 | Oleyl Erucate | | 100% | --- | 0.3 | --- | --- |
| Almond Oil | Prunus Amygdalus Dulcis (Sweet Almond) Oil | | 100% | --- | --- | 0.2 | 0.25 |
| Keltrol CG-SFT | Xanthan Gum | | 100% | 1.0 | 1.0 | | |
| Perfume oil composition 1B | Fragrance | | 100% | 0.25 | 0.25 | 0.25 | 0.25 |
| Na-Benzoate | Sodium Benzoate | | 100% | 0.5 | 0.5 | 0.5 | 0.5 |
| Citric Acid | Citric Acid | | 50% | q.s. | q.s. | q.s. | q.s. |
| NaCl | Sodium Chloride | | 100% | q.s. | q.s. | q.s. | q.s. |
| Floral water | Floral water | | 100% | --- | --- | --- | 10,0 |
| Water | Water | | 100% | 62.8 | 50.2 | 55.1 5 | 24.2 |
| | | | | | | | |
| pH value | | | | 5.1 | 5.0 | 5.3 | 4.8 |
| Viscosity [mPas] | | | | 6200 | 4600 | 680 0 | 5800 |
| Density | g/cm³ | | | | | | 1.02 8 |

Shampoo 49 corresponds to shampoo 1, where the perfume oil composition 1B is replaced by the same amount of the perfume oil composition 7.

Shampoo 50 corresponds to shampoo 2, where the perfume oil composition 1B is replaced by the same amount of the perfume oil composition 7.

Shampoo 51 corresponds to shampoo 3, where the perfume oil composition 1B is replaced by the same amount of the perfume oil composition 7.

Shampoo 52 corresponds to shampoo 4, where the perfume oil composition 1B is replaced by the same amount of the perfume oil composition 7.

Shampoo 53 corresponds to shampoo 5, where the perfume oil composition 1B is replaced by the same amount of the perfume oil composition 7.

Shampoo 54 corresponds to shampoo 6, where the perfume oil composition 1B is replaced by the same amount of the perfume oil composition 7.

Shampoo 55 corresponds to shampoo 7, where the perfume oil composition 1B is replaced by the same amount of the perfume oil composition 7.

Shampoo 56 corresponds to shampoo 8, where the perfume oil composition 1B is replaced by the same amount of the perfume oil composition 7.

Shampoo 105 corresponds to shampoo 1, where the perfume oil composition 1B is replaced by the same amount of the perfume oil composition 14.

Shampoo 106 corresponds to shampoo 2, where the perfume oil composition 1B is replaced by the same amount of the perfume oil composition 14.

Shampoo 107 corresponds to shampoo 3, where the perfume oil composition 1B is replaced by the same amount of the perfume oil composition 14.

Shampoo 108 corresponds to shampoo 4, where the perfume oil composition 1B is replaced by the same amount of the perfume oil composition 14.

Shampoo 109 corresponds to shampoo 5, where the perfume oil composition 1B is replaced by the same amount of the perfume oil composition 14.

Shampoo 110 corresponds to shampoo 6, where the perfume oil composition 1B is replaced by the same amount of the perfume oil composition 14.

Shampoo 111 corresponds to shampoo 7, where the perfume oil composition 1B is replaced by the same amount of the perfume oil composition 14.

Shampoo 112 corresponds to shampoo 8, where the perfume oil composition 1B is replaced by the same amount of the perfume oil composition 14.

**Table 18: Shower bath 1, 2, 3 and 4**

| **Shower bath** | | | | **1** | **2** | **3** | **4** |
|---|---|---|---|---|---|---|---|
| **Component** | **INCI** | | **Conc.** | **% b.w.** | | | |
| Plantapon SF | Sodium Cocoamphoacetate (and) Glycerin (and) Lauryl Glucoside (and) Sodium Cocoyl Glutamate (and) | 10-20 | 42 - 52% | 45.0 | 50.0 | 48.0 | 44.0 |
| | | 5-15 | | | | | |
| | | 5-15 | | | | | |
| | Sodium Lauryl Glucose Carboxylate water | 5 | | | | | |
| | | 5 | | | | | |
| | | 48 - 58 | | | | | |
| Lamesoft PO65 | Coco-Glucoside (and) Glyceryl Oleate water | 15-25 | 28,5 - 34% | 2.0 | 2.0 | 2.0 | 3.0 |
| | | 10-20 | | | | | |
| | | 62 - 67 | | | | | |
| Euperlan Green | Lauryl Glucoside (and) Stearyl Citrate water | 15 - 25 | 38 - | - | - | - | 2.0 |
| | | 15-25 | 44% | | | | |
| | | 55 - 65 | | | | | |
| Gluadin WLM Benz | Hydrolyzed Wheat Protein | | 21 - 24% | - | - | 1.0 | 0.5 |
| Keltrol CG-SFT | Xanthan Gum | | 100% | 1.0 | 0.5 | - | - |
| PLANTACARE^{®} PS 10 | Sodium Laureth Sulfate (and) Lauryl Polyglucose | | 33% | 4.5 | 6.0 | 10.5 | 7.5 |
| Copherol1250 C | Tocopheryl Acetate | | 100% | 0.1 | - | - | - |
| Cetiol J 600 | Oleyl Erucate | | 100% | - | 0.2 | - | - |
| Almond Oil | Prunus Amygdalus Dulcis (Sweet Almond) Oil | | 100% | - | - | 0.15 | 0.1 |
| Perfume oil composition 1B | Fragrance | | 100% | 0.25 | 0.25 | 0.25 | 0.25 |
| Na-Benzoate | Sodium Benzoate | | 100% | 0.5 | 0.5 | 0.5 | 0.5 |
| Citric Acid | Citric Acid | | 50% | q.s. | q.s. | q.s. | q.s. |
| Water | Water | | 100% | 46.65 | 40.55 | 37.6 | 42.15 |
| pH value | | | | 5.1 | 4.7 | 4.9 | 4.9 |
| Viscosity [mPas] | | | | 4200 | 4600 | 2900 | 2800 |
| Density | g/cm³ | | | | | | 1.039 |

**Table 19: Shower bath 5, 6, 7 and 8**

| **Shower bath** | | | **5** | **6** | **7** | **8** |
|---|---|---|---|---|---|---|
| **Component** | **INCI** | **Conc.** | **% b.w.** | | | |
| Texapon N70 | Sodium Laureth Sulfate | 70% | 12.9 | 12.9 | 14.3 | 14.3 |
| Dehyton PK45 | Cocam idopropyl Betaine | 44 - 46% | 5.4 | 5.4 | 5.4 | 5.4 |
| Plantacare 818UP | Coco-Glucoside | 51 - 53% | 2.0 | 2.0 | --- | --- |
| PLANTACARE^{®} PS 10 | Sodium Laureth Sulfate (and) Lauryl Polyglucose | 33% | 9.0 | 9.0 | 10.5 | 10.5 |
| DC 245 | Cyclopentasiloxane | 100% | 1.0 | --- | 1.1 | --- |
| Synfluid PAO2 | Hydrogenated Didecene | 100% | --- | 0.8 | --- | 1.0 |
| Jaguar C162 | Hydroxypropyl Guar Hydroxypropyltrimonium Chloride | 100% | 0.2 | 0.2 | --- | --- |
| AquaCAT CG518 | Guar Hydroxypropy-Itrimonium Chloride | 10% | --- | --- | 2.5 | 2.5 |
| Arlypon TT | PEG/PPG-120/10 Trimethylolpropane Trioleate (and) Laureth-2 | 40 - 50% | 1.0 | 1.5 | 1.0 | 1.5 |
| | | 40 - 50% | | | | |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 67.7 | 67.4 | 64.4 | 64.0 |
| pH value | | | 5.0 | 4.9 | 5.2 | 5.1 |
| Viscosity [mPas] | | | 6600 | 8100 | 6900 | 9300 |

Shower bath 49 corresponds to shower bath 1, where the perfume oil composition 1B is replaced by the same amount of the perfume oil composition 7.

Shower bath 50 corresponds to shower bath 2, where the perfume oil composition 1B is replaced by the same amount of the perfume oil composition 7.

Shower bath 51 corresponds to shower bath 3, where the perfume oil composition 1B is replaced by the same amount of the perfume oil composition 7.

Shower bath 52 corresponds to shower bath 4, where the perfume oil composition 1B is replaced by the same amount of the perfume oil composition 7.

Shower bath 53 corresponds to shower bath 5, where the perfume oil composition 1B is replaced by the same amount of the perfume oil composition 7.

Shower bath 54 corresponds to shower bath 6, where the perfume oil composition 1B is replaced by the same amount of the perfume oil composition 7.

Shower bath 55 corresponds to shower bath 7, where the perfume oil composition 1B is replaced by the same amount of the perfume oil composition 7.

Shower bath 56 corresponds to shower bath 8, where the perfume oil composition 1B is replaced by the same amount of the perfume oil composition 7.

Shower bath 105 corresponds to shower bath 1, where the perfume oil composition 1B is replaced by the same amount of the perfume oil composition 14.

Shower bath 106 corresponds to shower bath 2, where the perfume oil composition 1B is replaced by the same amount of the perfume oil composition 14.

Shower bath 107 corresponds to shower bath 3, where the perfume oil composition 1B is replaced by the same amount of the perfume oil composition 14.

Shower bath 108 corresponds to shower bath 4, where the perfume oil composition 1B is replaced by the same amount of the perfume oil composition 14.

Shower bath 109 corresponds to shower bath 5, where the perfume oil composition 1B is replaced by the same amount of the perfume oil composition 14.

Shower bath 110 corresponds to shower bath 6, where the perfume oil composition 1B is replaced by the same amount of the perfume oil composition 14.

Shower bath 111 corresponds to shower bath 7, where the perfume oil composition 1B is replaced by the same amount of the perfume oil composition 14.

Shower bath 112 corresponds to shower bath 8, where the perfume oil composition 1B is replaced by the same amount of the perfume oil composition 14.

**Table 20: Hydro-alcoholic AP/Deo pump spray**

| **Component** | | **INCI** | **amount %** |
|---|---|---|---|
| Ethanol, cosm. | Alcohol | | 40.0 |
| water, demin. | Aqua | | 37.5 |
| Hydagen CAT | Triethyl Citrate | | 2.0 |
| Hydagen DCMF | Chitosan | | 10.0 |
| Eumulgin^{®} HRE 60 | PEG-60 Hydrogenated Castor Oil | | 1.5 |
| Chlorhydrol (50% solut.) | Aluminium Chlorohydrate | | 8.0 |
| Perfume oil composition 1B | | | 1.0 |

Deo pump spray according to table 20, where the perfume oil composition 1B is replaced by the same amount of the perfume oil composition 7.

Deo pump spray according to table 20, where the perfume oil composition 1B is replaced by the same amount of the perfume oil composition 14.

**Table 21: Aerosol 1**

| **Component** | **INCI** | **amount %** |
|---|---|---|
| Ethanol, cosm. | Alcohol | 80.0 |
| water, demin. | Aqua | 16.7 |
| Propylene glycol | Propylene Glycol | 1.0 |
| Eumulgin^{®} HRE 60 | PEG-60 Hydrogenated Castor Oil | 1.5 |
| alpha-Bisabolol | Bisabolol | 0.05 |
| Triclosan | Triclosan | 0.25 |
| Perfume oil composition 1B | | 1.0 |

Aerosol 7 corresponds to aerosol 1, where the perfume oil composition 1B is replaced by the same amount of the perfume oil composition 7.

Aerosol 14 corresponds to aerosol 1, where the perfume oil composition 1B is replaced by the same amount of the perfume oil composition 14.

**Table 22: Aqueous/alcoholic AP/Deo roll-on**

| **Component** | **INCI** | **amount %** |
|---|---|---|
| Ethanol, cosm. | Alcohol | 40.0 |
| water, demin. | Aqua | 37.0 |
| Hydagen CAT | Triethyl Citrate | 2.0 |
| Hydagen DCMF | Chitosan | 10.0 |
| Eumulgin^{®} HRE 60 | PEG-60 Hydrogenated Castor Oil | 1.5 |
| Chlorhydrol (50% solut.) | Aluminium Chlorohydarte | 8.0 |
| Klucel M | Hydroxypropylcellulose | 0.5 |
| Perfume oil composition 1B | | 1.0 |

Aqueous/alcoholic AP/Deo roll-on according to table 22, where the perfume oil composition 1B is replaced by the same amount of the perfume oil composition 7. Aqueous/alcoholic AP/Deo roll-on according to table 22, where the perfume oil composition 1B is replaced by the same amount of the perfume oil composition 14.

**Table 23: Styling Gel Type "Out of Bed"**

| **Phase** | **Component** | **INCI** | **%** | **Function** |
|---|---|---|---|---|
| I | Alcool 96% (Prolabo) | Alcohol 96% | 12.00 | Solvent |
| | Luviskol VA 64 (BASF ) | PVP/VA | 4.50 | Styling agent |
| | Polyox WSR-301 | PEG-90M | 0.25 | Styling agent |
| | Methocel E4M Premium EP (DOW) | Hydroxypropyl Methylcellulose | 0.60 | Thickener |
| | DEHYQUART^{®} L 80 | Dicocoylethyl Hydroxyethylmonium Methosulfate (and) Propylene Glycol | 0.60 | Active agent |
| II. | Eumulgin^{®} HRE 60 | Hardened castor oil with approx. 60 mol EO | 1.00 | Solubilizer |
| | CETIOL^{®} OE | Dicaprylyl Ether | 1.50 | Emollient |
| III. | Water, de-ionized | Aqua | a.d. | |
| IV. | HISPAGEL^{®} 200 | Glycerin (and) Glyceryl Polyacrylate | 36.70 | Thickener |
| V. | Perfume oil composition 1B | | 0.02 | |
| >> | pH-value | | 6.8 | |
| >> | Viscosity (mPas) Brookfield 10rpm | RVT 23°C spindle 5, | 220.000 mPas | |

Styling gel according to table 23, where the perfume oil composition 1B is replaced by the same amount of the perfume oil composition 7.

Styling gel according to table 23, where the perfume oil composition 1B is replaced by the same amount of the perfume oil composition 14.

**Table 24: Shaving Foam**

| **Phase** | **Component** | **INCI** | **%** | **Function** |
|---|---|---|---|---|
| | CUTINA^{®} FS 45 | Stearic Acid (and) Palmitic Acid | 7.6 | Surfactant (foam consistency) |
| | PLANTACARE^{®} 1200 UP | Lauryl Glucoside | 6.0 | Surfactant (inhibitory effect) |
| | MONOMULS^{®} 90-O 18 | Glyceryl Oleate | 1.2 | W/O emulsifier (moisturizing) |
| | EUMULGIN^{®} O20 S | Oleth-20 | 1.2 | O/W-emulsifier (stability) |
| | CETIOL^{®} CC | Dicaprylyl Carbonate | 2.0 | Emollient (low viscosity) |
| | DEHYQUART^{®} SP | Quaternium-52 | 0.5 | Surfactant (inhibitor) |
| | TEA (99%) | | 4.0 | Neutralizer (for Cutina FS 45) |
| | Glycerin | Glycerin | 3.0 | Humectant |
| | Propylene Glycol | Propylene Glycol | 3.0 | Humectant (low viscosiy) |
| | Emulgin^{®} HRE 60 | Hardened castor oil with approx. 60 mol EO | 2.0 | Solubilizer |
| | D-Panthenol | Panthenol | 0.2 | Care additive |
| | Phenonip | Phenoxyethanol (and) Parabens | 0.5 | Preservative |
| | Perfume oil composition 1B | | 0.3 | Fragrance |
| | Distilled or Deionized Water | Aqua | ad 100 | |

Shaving foam according to table 24, where the perfume oil composition 1B is replaced by the same amount of the perfume oil composition 7.

Shaving foam according to table 24, where the perfume oil composition 1B is replaced by the same amount of the perfume oil composition 14.

**Table 25: Sensitive skin Baby shampoo**

| **Ingredients** | | **INCI / Chemistry** | **%** | **Function** |
|---|---|---|---|---|
| I. | PLANTAPON^{®} LGC SORB | Sodium Lauryl Glucose Carboxylate (and) Lauryl Glucoside | 28.00 | Surfactant, cleaning |
| | ARLYPON^{®} TT | PEG/PPG-120/10 Trimethylolpropane Trioleate (and) Laureth-2 | 2.00 | Thickener |
| | DEHYTON^{®} MC | Sodium Cocoamphoacetate | 6.00 | Surfactant |
| | EUMULGIN^{®} SML 20 | Polysorbate 20 | 3.00 | Solubilizer |
| | LAMESOFT^{®} PO 65 | Coco-Glucoside (and) Glyceryl Oleate | 2.20 | Lipid layer enhancer |
| | MELHYDRAN^{™} LS 4420 | Honey Extract | 1.00 | Active ingredient |
| | Perfume oil composition 1B | Perfume | 0.20 | Perfume |
| | Dermosoft 1388 (Dr. Straetmans) | Water (and) Glycerin (and) Sodium Levulinate (and) Sodium Anisate | 4.00 | Active ingredient |
| | Water, demin. | Aqua | 53.60 | |

Sensitive skin baby shampoo according to table 25, where the perfume oil composition 1B is replaced by the same amount of the perfume oil composition 7.

Sensitive skin baby shampoo according to table 25, where the perfume oil composition 1B is replaced by the same amount of the perfume oil composition 14.

**Table 26: Body wash for Sensitive Skin**

| **Ingredients** | | **INCI / Chemistry** | **%** | **Function** |
|---|---|---|---|---|
| I. Water, demin. | | Aqua | 70.90 | |
| | TEXAPON^{®} N 70 | Sodium Laureth Sulfate | 13.00 | Surfactant |
| | PLANTACARE^{®} 818 UP | Coco-Glucoside | 3.00 | Surfactant |
| | LAMESOFT^{®} PO 65 | Coco-Glucoside (and) Glyceryl Oleate | 5.00 | Active, skin conditioning |
| | Perfume oil composition 1B | Fragrance | 0.15 | Perfume |
| | Sodium Benzoate | Sodium Benzoate | 0.55 | Preservative |
| II. | DEHYQUART^{®} CC7 BZ | Polyquaternium-7 | 2.00 | Active, hair conditioning |
| III. | MELHYDRAN^{™} LS 4420 | Honey Extract | 1.00 | Active, moisturi- |
| | | | | zing |
| | ARLYPON^{®} TT | PEG/PPG-120/10 Trimethylolpropane Trioleate (and) Laureth-2 | 0.50 | Thickener |
| | DEHYTON^{®} PK 45 | Cocamidopropyl Betaine | 3.80 | Surfactant |
| IV. | Sodium Chloride | Sodium Chloride | 0.10 | Agent, thickening |

Body wash for sensitive skin according to table 26, where the perfume oil composition 1B is replaced by the same amount of the perfume oil composition 7.

Body wash for sensitive skin according to table 26, where the perfume oil composition 1B is replaced by the same amount of the perfume oil composition 14.

**Table 27: Gloss Enhancing Shampoo for Sensitive Scalp**

| **Ingredients** | | **INCI** / **Chemistry** | **%** | **Function** |
|---|---|---|---|---|
| I. | Water, demin. | Aqua | 71.60 | |
| | Luviquat Ultra Care (BASF) | Polyquaternium-44 | 1.50 | Active, hair conditioning |
| II. | Sodium Benzoate | Sodium Benzoate | 0.55 | Preservative |
| III. | TEXAPON^{®} N 70 | Sodium Laureth Sulfate | 14.30 | Surfactant |
| | DEHYTON^{®} PK 45 | Cocamidopropyl Betaine | 5.40 | Surfactant |
| | MELHYDRAN^{™} LS 4420 | Honey Extract | 1.00 | Active, moisturizing |
| | Perfume oil composition 1B | Fragrance | 0.15 | Perfume |
| IV. | DEHYDOL^{®} LS 2 DEO N | Laureth-2 | 1.00 | Thickener |
| V. | LAMESOFT^{®} CARE | PEG-4 Distearyl Ether (and) Sodium Laureth sulfate (and) Distearyl Ether (and) Dicaprylyl Ether | 3.00 | Active, hair conditioning |
| VI. | Sodium Chloride | Sodium Chloride | 1.50 | Agent, thickening |

Gloss enhancing shampoo for sensitive scalp according to table 27, where the perfume oil composition 1B is replaced by the same amount of the perfume oil composition 7. Gloss enhancing shampoo for sensitive scalp according to table 27, where the perfume oil composition 1B is replaced by the same amount of the perfume oil composition 14.

**Table 28: Deo Stick**

| **Ingredients** | | **INCI** / **Chemistry** | **%** | **Function** |
|---|---|---|---|---|
| I. | LANETTE^{®} 18 DEO | Stearyl Alcohol | 18.50 | Consistency factor |
| | CUTINA^{®} HR POWDER | Hydrogenated Castor Oil | 4.50 | Consistency factor |
| | CUTINA^{®} CP | Cetyl Palmitate | 25.00 | Emollient |
| | CETIOL^{®} MM | Myristyl Myristate | 10.00 | Emollient |
| | CETIOL^{®} RLF | Caprylyl Caprylate/Caprate | 5.00 | Emollient |
| | MYRITOL^{®} 312 | Caprylic/Capric Triglyceride | 29.30 | Emollient |
| | HYDAGEN^{®} C.A.T. | Triethyl Citrate | 5.00 | Active, antiperspirant |
| II. | COSMEDIA^{®} SILC | Silica | 2.00 | Skin feel modifier |
| | PHYTOSOOTHE^{™} LS 9766 | Brassica Campestris (Rapeseed) Sterols (and) Cetearyl Alcohol | 0.50 | Active ingredient |
| III. | Perfume oil composition 1B | Fragrance | 0.20 | Perfume |

Deo stick according to table 28, where the perfume oil composition 1B is replaced by the same amount of the perfume oil composition 7.

Deo stick according to table 28, where the perfume oil composition 1B is replaced by the same amount of the perfume oil composition 14.

**Table 29: Baby Wipe**

| **Ingredients** | | **INCI** / **Chemistry** | % | **Function** |
|---|---|---|---|---|
| I. | Water, demin. | Water | 95.25 | |
| | Sodium Benzoate | Sodium Benzoate | 0.50 | Preservative |
| II. | EMULGADE^{®} CPE | Vegetable Oil (and) Glycerin (and) Lauryl Glucoside (and) Polyglyceryl-2-Dipolyhydroxystearate (and) Glyceryl Oleate | 4.00 | Emulsion base (O/W) |
| | LIPOFRUCTYL^{™} ARGAN LS 9779 | Argania Spinosa Kernel Oil | 0.10 | Active ingredient |
| | Perfume oil composition 1B | Fragrance | 0.10 | Perfume |
| III. | Citric Acid (50 %) | Citric Acid | 0.15 | Agent, pH adjusting |

Baby wipe according to table 29, where the perfume oil composition 1B is replaced by the same amount of the perfume oil composition 7.

Baby wipe according to table 29, where the perfume oil composition 1B is replaced by the same amount of the perfume oil composition 14.

**Table 30: After shave balm**

| **Ingredients** | | **INCI** | **%** | **Function** |
|---|---|---|---|---|
| I | Myritol^{®} 331 | Cocoglycerides | 2.50 | Emollient |
| | Cetiol^{®} Sensoft | Propylheptyl Caprylate | 5.00 | Emollient |
| | Cetiol^{®} C5 | Coco-Caprylate | 2.50 | Emollient |
| | Cosmedia^{®} Triple C | Polyquaternium-37 (and) Dicaprylyl Carbonate (and) Lauryl Glucoside | 1.50 | Cationic Polymer |
| II | Deionized Water | Aqua | 84.80 | |
| | Glycerine | Glycerin | 1.00 | Humectant |
| III | Anasensyl^{™} LS 9322 | Mannitol (and) Ammonium Glycyrrhizate (and) Caffeine (and) Zinc Gluconate (and) Aesculus Hippocastanum Extract | 1.00 | Soothing Active |
| IV | Perfume oil composition 1B | Fragrance | 0.10 | Perfume |
| V | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| | VI NaOH (Aq. Sol. 25%) | Sodium Hydroxide | 0.10 | Neutralizing Agent |

After shave balm according to table 30, where the perfume oil composition 1B is replaced by the same amount of the perfume oil composition 7.

After shave balm according to table 30, where the perfume oil composition 1B is replaced by the same amount of the perfume oil composition 14.

**Table 31: Face Gel**

| **Ingredients** | | **INCI** | **%** | **Function** |
|---|---|---|---|---|
| I | Deionized Water | Aqua | 71.65 | |
| | Glycerine | Glycerin | 3.00 | Humectant |
| | EDETA B Powder (BASF) | Tetrasodium EDTA | 0.05 | Complexing Agent |
| II | Cosmedia^{®} SP | Sodium Polyacrylate | 1.20 | Emulsifying Polymer |
| III | Cegesoft^{®} PFO | Passiflora Incarnata Oil | 2.00 | Emollient |
| | Uvinul MC 80 (BASF) | Ethylhexyl Methoxycinnamate | 2.00 | UVB Filter |
| | KF 96, 100 cs (Shin Etsu) | Dimethicone | 2.00 | Emollient |
| IV | Cetiol^{®} CC | Dicaprylyl Carbonate | 5.00 | Emollient |
| | Cetiol^{®} C5 | Coco-Caprylate | 4.00 | Emollient |
| | Uvinul A Plus Granular (BASF) | Diethylamino Hydroxybenzoyl Hexyl Benzoate | 0.50 | UVA Filter |
| | Cegesoft^{®} SBE | Butyrospermum Parkii | 1.50 | Emollient |
| | Eumulgin^{®} SG | Sodium Stearoyl Glutamate | 0.10 | O/W Emulsifier |
| V | Deionized Water | Aqua | 5.00 | |
| | Skinasensyl^{™} PW LS 9852 | Mannitol (and) Sodium Citrate (and) Acetyl Tetrapeptide-15 | 0.30 | Soothing Active |
| VI | Covi-ox^{®} T70C | Tocopherol | 0.10 | Antioxidant |
| | Perfume oil composition 1B | Fragrance | 0.10 | Perfume |
| VII | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face gel according to table 31, where the perfume oil composition 1B is replaced by the same amount of the perfume oil composition 7.

Face gel according to table 31, where the perfume oil composition 1B is replaced by the same amount of the perfume oil composition 14.

**Table 32: Face Day Care Cream**

| **Ingredients** | | **INCI** | **%** | **Function** |
|---|---|---|---|---|
| I | Deionized Water | Aqua | 69.55 | |
| | Glycerine | Glycerin | 3.00 | Humectant |
| | EDETA B Powder (BASF) | Tetrasodium EDTA | 0.05 | Complexing Agent |
| II | Cosmedia^{®} SP | Sodium Polyacrylate | 0.80 | Emulsifying Polymer |
| III | Emulgade^{®} PL 68/50 | Cetearyl Glucoside (and) Cetearyl Alcohol | 2.00 | Self-Emulsifying Base |
| | Eumulgin^{®} SG | Sodium Stearoyl Glutamate | 0.50 | O/W Emulsifier |
| | Monomuls^{®} 90-0-18 | Glyceryl Oleate | 2.00 | W/O Emulsifier |
| | Cutina^{®} GMS-V | Glyceryl Stearate | 2.00 | Consistency Giving Factor |
| | Cetiol^{®} Sensoft | Propylheptyl Caprylate | 5.00 | Emollient |
| | Myritol^{®} 331 | Cocoglycerides | 3.00 | Emollient |
| | Cegesoft^{®} PFO | Passiflora Incarnata Oil | 3.00 | Emollient |
| | Lipodermol^{™} LS 8656 | Octyldodecanol (and) Lecithin (and) Arachidyl Propionate (and) Tocopheryl Acetate (and) Retinyl Palmitate (and) Ethyl Linoleate (and) Ethyl Linolenate (and) Ethyl | 1.00 | Anti-Ageing Active |
| | General^{®} R | Oleate Brassica Campestris (Rapeseed) Sterols | 1.00 | Anti-inflammatory Active |
| IV | Sphingoceryl^{™} WS LS 9859 | Aqua (and) Glycerin (and) Helianthus Annuus Seed Extract (and) Decyl Glucoside | 1.00 | Moisturizing Active |
| | Deionized Water | Aqua | 4.00 | |
| | Perfume oil composition 1B | Fragrance | 0.10 | Perfume |
| V | Covi-ox^{®} T70C | Tocopherol | 0.50 | Antioxidant |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face day care cream according to table 32, where the perfume oil composition 1B is replaced by the same amount of the perfume oil composition 7.

Face day care cream according to table 32, where the perfume oil composition 1B is replaced by the same amount of the perfume oil composition 14.

**Table 33: Face Cleanser**

| **Ingredients** | | **INCI** | **%** | **Function** |
|---|---|---|---|---|
| I | Deionized Water | Aqua | 80.15 | |
| | EDETA B Powder (BASF) | Tetrasodium EDTA | 0.05 | Complexing Agent |
| | Eumulgin^{®} SG | Sodium Stearoyl Glutamate | 0.30 | O/W Emulsifier |
| II | Cosmedia^{®} SP | Sodium Polyacrylate | 1.20 | Emulsifier |
| III | Isopropylpalmitate | Isopropyl palmitate | 5.00 | Emollient |
| | Cetiol^{®} CC | Dicaprylyl Carbonate | 3.00 | Emollient |
| | Cetiol^{®} C5 | Coco-Caprylate | 7.00 | Emollient |
| IV | Plantacare^{®} 818 UP | Coco Glucoside | 0.75 | Non Ionic Surfactant |
| V | Indinyl^{®} CA LS 8998 | Water (and) Cassia Angustifolia Seed Polysaccharide | 0.50 | Smoothing and Moisturizing Active |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| VII | NaOH (Aq. Sol. 25%) | Sodium Hydroxide | 0.45 | Neutralizing Agent |

Face cleanser according to table 33, where the perfume oil composition 1B is replaced by the same amount of the perfume oil composition 7.

Face cleanser according to table 33, where the perfume oil composition 1B is replaced by the same amount of the perfume oil composition 14.

**Table 34: Sun Care SPF50+, Sprayable Lotion**

| **Ingredients** | | **INCI / Chemistry** | **%** | **Function** |
|---|---|---|---|---|
| I. | DEHYMULS^{®} PGPH | Polyglyceryl-2 Dipolyhydroxystearate | 4.00 | Emulsifier (W/O) |
| | CETIOL^{®} B | Dibutyl Adipate | 8.00 | Emollient |
| | MYRITOL^{®} 331 | Cocoglycerides | 5.00 | Emollient |
| | Tinosorb S (BASF) | Bis-Ethylhexyloxyphenol Methoxyphenyl Triazine | 3.00 | UV filter, UV-A + UV-B |
| | Uvinul T 150 ( BASF) | Ethylhexyl Triazone | 2.50 | UV filter, UV-B |
| | Uvinul A Plus (BASF) | Diethylamino Hydroxybenzoyl Hexyl Benzoate | 10.00 | UV filter, UV-A |
| | Uvinul MC 80 (BASF) | Ethylhexyl Methoxycinnamate | 10.00 | UV filter, UV-B |
| | Sensiva SC 50 (Schülke) | Ethylhexylglycerin | 0.50 | Preservative |
| II. | Water, demin. | Water | 45.55 | |
| | Glycerin | Glycerin | 3.00 | Agent, humectant |
| | EDETA BD (BASF) | Disodium EDTA | 0.05 | Chelating agent |
| | Euxyl PE 9010 (Schülke) | Phenoxyethanol, Ethylhexylglycerin | 1.00 | Preservative |
| | Keltrol CG-T (CP Kelco) | Xanthan Gum | 0.10 | Agent, thickening |
| | Veegum Ultra (RT | Magnesium Aluminum | 2.00 | Stabilizer |
| | Vanderbilt, Inc.) | Silicate | | |
| III. | PLANTAPON^{®} LGC SORB | Sodium Lauryl Glucose Carboxylate (and) Lauryl Glucoside | 1.50 | Surfactant |
| IV. | Eusolex T 2000 (Merck) | Titanium Dioxide and Alumina and Simethicone | 3.5 | UV filter, UV-A + UV-B |
| V. | DN-AGE^{™} PW LS 9827 | Cassia Alata Leaf Extract (and) Maltodextrin | 0.10 | Active ingredient |
| | Perfume oil composition 1B | Fragrance | 0.20 | Perfume |

Sun Care SPF50+, sprayable lotion according to table 34, where the perfume oil composition 1B is replaced by the same amount of the perfume oil composition 7.

Sun Care SPF50+, sprayable lotion according to table 34, where the perfume oil composition 1B is replaced by the same amount of the perfume oil composition 14.

**Table 35: Hand dish cleaner, regular**

| **Hand dish cleaner, regular** | | |
|---|---|---|
| **Component** | **INCI** | **Amount %** |
| Lutensit ^{®} A-LBN 50 | Benzenesulfonic acid, C10-13-alkyl derivs., sodium salts | 25 |
| Lutensol ^{®} GD 70 | Alkyl polyglucoside | 3 |
| Lutensol ^{®} A 7 N | Fatty alcohol ethoxylate | 2 |
| Perfume oil composition 1B | Fragrance | 0.7 |
| Dyes | Dye | 0.05 |
| Water, de ionized | Aqua | ad 100 |

Hand dish cleaner according to table 35, where the perfume oil composition 1B is replaced by the same amount of the perfume oil composition 7.

Hand dish cleaner according to table 35, where the perfume oil composition 1B is replaced by the same amount of the perfume oil composition 14.

**Table 36: Body lotion**

| **Ingredients** | **INCI** | **%** | **Function** |
|---|---|---|---|
| I Eumulgin^{®} SG | Sodium Stearoyl Glutamate | 0.40 | O/W Emulsifier |
| Cutina^{®} GMS-V | Glyceryl Stearate | 1.50 | Consistency Giving Factor |
| Cetiol^{®} SB 45 | Butyrospermum Parkii (Shea) Butter | 3.00 | Emollient |
| Cetiol^{®} C5 | Coco-Caprylate | 2.00 | Emollient |
| Irwinol^{™} LS 9890 | Octyldodecanol (and) Irvingia Gabonensis (and) Hydrogenated CocoGlycerides | 1.00 | Moisturizing Active |
| II Water, demin. | Aqua | 86.77 | |
| Glycerine | Glycerin | 3.00 | Humectant |
| EDETA B Powder (BASF) | Tetrasodium EDTA | 0.05 | Complexing Agent |
| III Rheocare^{™} C Plus | Carbomer | 0.35 | Thickener |
| IV NaOH (Aq sol. 25%) | Sodium Hydroxide | 0.10 | Neutralizing Agent |
| V Covi-ox^{®} T70C | Tocopherol | 0.05 | Antioxidant |
| Perfume oil composition 1B | Fragrance | 0.10 | Perfume |
| VI Euxyl PE 9010 (Schülke) | Phenoxyethanol (and) Ethylhexylglycerin | 1.00 | Preservative |
| Sensiva SC50 (Schülke) | Ethylhexylglycerin | 0.50 | Preservative |
| VII NaOH (Aq sol. 25%) | Sodium Hydroxide | 0.18 | Neutralizing Agent |

Body lotion according to table 36, where the perfume oil composition 1B is replaced by the same amount of the perfume oil composition 7.

Body lotion according to table 36, where the perfume oil composition 1B is replaced by the same amount of the perfume oil composition 14.

**Table 35: Hand dish cleaner, concentrate:**

| **Hand dish cleaner, concentrate** | | |
|---|---|---|
| **Component** | **INCI** | **amount %** |
| | | |
| Lutensit ^{®} A-LBN 50 | Benzenesulfonic acid, C10-13-alkyl derivs., sodium salts | 40 |
| Lutensit ^{®} AS 2230 | Alkylether sulphate | 25 |
| Lutensol ^{®} GD 70 | Alkyl polyglucoside | 7 |
| Lutensol ^{®} A 7 N | Fatty alcohol ethoxylate | 4 |
| Perfume oil composition 1B | Perfume | 1 |
| Dyes | Dye | 0.05 |
| Water, de ionized | Aqua | ad 100 |

Hand dish cleaner according to table 37, where the perfume oil composition 1B is replaced by the same amount of the perfume oil composition 7.

Hand dish cleaner according to table 37, where the perfume oil composition 1B is replaced by the same amount of the perfume oil composition 14.

**Table 38: Sanitary cleaner, concentrate**

| **Sanitary cleaner, concentrate** | | |
|---|---|---|
| **Component** | **INCI** | **amount %** |
| Lutensol ^{®} TO 8 | isotridecanolethoxylate | 5 |
| Phosphoric acid | Phosphoric acid | 20 |
| Korantin ^{®} PM | 2-propyn-1-ol, ethoxylated | 3 |
| Citric acid | Citric acid | 3 |
| Perfume oil composition 1B | Fragrance | 1 |
| Water, deionized | Aqua | ad 100 |

Sanitary cleaner according to table 38, where the perfume oil composition 1B is replaced by the same amount of the perfume oil composition 7.

Sanitary cleaner according to table 38, where the perfume oil composition 1B is replaced by the same amount of the perfume oil composition 14.

**Table 39: All purpose cleaner**

| **All purpose cleaner** | | |
|---|---|---|
| **Component** | **INCI** | **amount %** |
| Lutensit ^{®} A LBA | Benzenesulfonic acid, 4-C10-13-sec-alkyl derivs | 1 |
| Ammonia | Ammonia | 0.2 |
| Lutensol ^{®} CS 6250 | Hexan-1-ol, ethoxylated | 3 |
| Trilon ^{®} A | trisodium nitrilotriacetate | 1 |
| Citric acid | Citric acid | 0.65 |
| Ethanol | Ethanol | 5 |
| Perfume oil composition 1B | Perfume | 0.5 |
| Water, de ionized | Aqua | ad 100 |

All purpose cleaner according to table 39, where the perfume oil composition 1B is replaced by the same amount of the perfume oil composition 7.

All purpose cleaner according to table 39, where the perfume oil composition 1B is replaced by the same amount of the perfume oil composition 14.

**Table 40: Anti bacterial fabric softener**

| **Anti-bacterial fabric softener** | | |
|---|---|---|
| **Component** | **INCI** | **amount %** |
| Tinosan ^{®} HP 100 | Hydroxydichlordiphenyl ether | 0.3 |
| Dehyquat AU-46 | Esterquat | 4 |
| Lutensol ^{®} AO 7 | Alcohols, C₁₃-₁₅, ethoxylated | 0.5 |
| Perfume oil composition 1B | Fragrance | 0.5 |
| Water, deionized | Aqua | ad 100 |

Anti bacterial fabric softener according to table 40, where the perfume oil composition 1B is replaced by the same amount of the perfume oil composition 7.

Anti bacterial fabric softener according to table 40, where the perfume oil composition 1B is replaced by the same amount of the perfume oil composition 14.

## Claims

1. The use of a compound of the formula 1.3 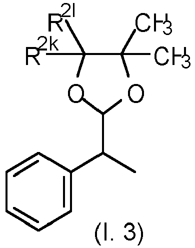 wherein
R2k and R^{2l} are methyl;
or of a stereoisomer thereof or of a mixture of stereoisomers thereof,
as an aroma chemical.

2. The use of the compound of formula 1.3 or of a stereoisomer or of a mixture of stereoisomers thereof as defined in claim 1, for modifying the scent character of a fragranced composition.

3. The use according to any of the preceding claims, in a composition selected from the group consisting of perfume compositions, body care compositions, hygiene articles, cleaning compositions, textile detergent compositions, foods, food supplements, pharmaceutical compositions and crop protection compositions.

4. Aroma chemical composition, comprising
- the compound of formula (1.3) or a stereoisomer thereof or a mixture of stereoisomers thereof according to claim 1; and
- a further aroma chemical and/or a non-aroma chemical carrier, where the non-aroma chemical carrier is in particular selected from the group consisting of surfactants, oil components and solvents.

5. The composition according to claim 4, selected from the group consisting perfume compositions, body care compositions, hygiene articles, cleaning compositions, textile detergent compositions, compositions for scent dispensers, foods, food supplements, pharmaceutical compositions and crop protection compositions.

6. The composition according to any of claims 4 or 5, comprising the compound of formula 1.3 or a stereoisomer thereof or a mixture of stereoisomers thereof in an overall amount of from 0.001 to 99.9% by weight, based on the total weight of the composition.

## Patentansprüche

1. Verwendung einer Verbindung der Formel I.3 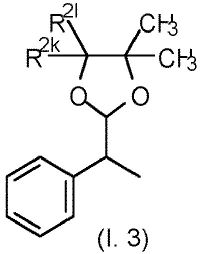 wobei
R^{2k} und R^{2l} für Methyl stehen;
oder eines Stereoisomers davon oder eines Gemischs von Stereoisomeren davon
als Aromachemikalie.

2. Verwendung der Verbindung der Formel I.3 oder eines Stereoisomers davon oder eines Gemischs von Stereoisomeren davon zur Modifizierung des Duftcharakters einer duftstoffhaltigen Zusammensetzung.

3. Verwendung nach einem der vorhergehenden Ansprüche in einer Zusammensetzung aus der Gruppe bestehend aus Parfümzusammensetzungen, Körperpflegezusammensetzungen, Hygieneartikeln, Reinigungszusammensetzungen, Textilwaschmittelzusammensetzungen, Lebensmitteln, Nahrungsergänzungsmitteln, pharmazeutischen Zusammensetzungen und Pflanzenschutzzusammensetzungen.

4. Aromachemikalienzusammensetzung, umfassend
- die Verbindung der Formel (I.3) oder ein Stereoisomer davon oder ein Gemisch von Stereoisomeren davon nach Anspruch 1 und
- eine weitere Aromachemikalie und/oder einen Nicht-Aromachemikalien-Träger, wobei der Nicht-Aromachemikalien-Träger insbesondere aus der Gruppe bestehend aus Tensiden, Ölkomponenten und Lösungsmitteln ausgewählt ist.

5. Zusammensetzung nach Anspruch 4, ausgewählt aus der Gruppe bestehend aus Parfümzusammensetzungen, Körperpflegezusammensetzungen, Hygieneartikeln, Reinigungszusammensetzungen, Textilwaschmittelzusammensetzungen, Zusammensetzungen für Duftspender, Lebensmitteln, Nahrungsergänzungsmitteln, pharmazeutischen Zusammensetzungen und Pflanzenschutzzusammensetzungen.

6. Zusammensetzung nach einem der Ansprüche 4 oder 5, umfassend die Verbindung der Formel (I.3) oder ein Stereoisomer davon oder ein Gemisch von Stereoisomeren davon in einer Gesamtmenge von 0,001 bis 99,9 Gew.-%, bezogen auf das Gesamtgewicht der Zusammensetzung.

## Revendications

1. Utilisation d'un composé de la formule I.3 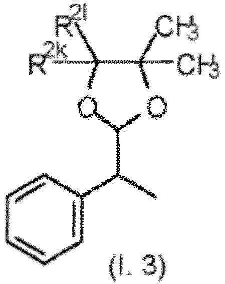 R^{2k} et R^{2l} étant méthyle ;
ou d'un stéréoisomère correspondant ou d'un mélange de stéréoisomères correspondants,
en tant que substance chimique d'arôme.

2. Utilisation du composé de la formule I.3 ou d'un stéréoisomère ou d'un mélange de stéréoisomères correspondants tel(s) que défini(s) dans la revendication 1, pour la modification du caractère parfumé d'une composition fragrancée.

3. Utilisation selon l'une quelconque des revendications précédentes, dans une composition choisie dans le groupe constitué par des compositions de parfum, des compositions de soin personnel, des articles d'hygiène, des compositions de nettoyage, des compositions de détergent pour textile, des produits alimentaires, des suppléments alimentaires, des compositions pharmaceutiques et des compositions de protection de cultures.

4. Composition chimique d'arôme, comprenant
- le composé de formule (I.3) ou un stéréoisomère ou un mélange de stéréoisomères correspondants selon la revendication 1 ; et
- une substance chimique d'arôme supplémentaire et/ou un support chimique non d'arôme, où le support chimique non d'arôme est en particulier choisi dans le groupe constitué par des tensioactifs, des composants d'huile et des solvants.

5. Composition selon la revendication 4, choisie dans le groupe constitué par des compositions de parfum, des compositions de soin personnel, des articles d'hygiène, des compositions de nettoyage, des compositions de détergent pour textile, des compositions pour diffuseurs de parfum, des produits alimentaires, des suppléments alimentaires, des compositions pharmaceutiques et des compositions de protection de cultures.

6. Composition selon l'une quelconque des revendications 4 et 5, comprenant le composé de formule (I.3) ou un stéréoisomère ou un mélange de stéréoisomères correspondants en une quantité globale allant d'environ 0,001 à 99,9 % en poids, sur la base du poids total de la composition.
